# EUROPEAN PATENT SPECIFICATION

(11) **EP 1 073 473 B1**
(45) Date of publication and mention of the grant of the patent: **17.10.2007**
(21) Application number: 99918133.2
(22) Date of filing: 22.04.1999
(51) Int. Cl.: A61K 49/00, A61K 41/00

(54) **IMPROVEMENTS IN OR RELATING TO CONTRAST AGENTS**
VERBESSERUNGEN IN ODER BEZÜGLICH ZU KONTRASTMITTEL
AMELIORATIONS APPORTEES A DES AGENTS DE CONTRASTE OU EN RAPPORT AVEC CES AGENTS

(30) Priority: 22.04.1998 GB 9808599
(43) Date of publication of application: 07.02.2001
(73) Proprietor: GE Healthcare AS, 0485 Oslo (NO)
(72) Inventor: ERIKSEN, Morten, GE Healthcare AS, N-0401 Oslo (NO); TOLLESHAUG, Helge, GE Healthcare AS, N-0401 Oslo (NO); SKURTVEIT, Roald, GE Healthcare AS, N-0401 Oslo (NO); CUTHBERTSON, Alan, GE Healthcare AS, N-0401 Oslo (NO); OSTENSEN, Jonny, GE Healthcare AS, N-0401 Oslo (NO); FRIGSTAD, Sigmund, N-7035 Trondheim (NO); RONGVED, Pal, N-1450 Nesoddtangen (NO)
(74) Representative: Flechsler, Insa
(86) International application number: PCT/GB1999/001221
(87) International publication number: WO 1999/053963

(56) References cited:
- WO-A-97/40858
- WO-A-98/17324
- WO-A-98/18495
- WO-A-98/18497
- WO-A-98/18498
- US-A- 5 228 446
- US-A- 5 580 575
- CAMINATI G ET AL: "Lipopeptides of myelin basic protein in mono- and multilayers" THIN SOLID FILMS, vol. 327-329, 31 August 1998 (1998-08-31), page 37-41 XP004151893 ISSN: 0040-6090
- ONO S ET AL: "INTERACTION OF AMPHIPATHIC MODEL LIPOPEPTIDES WITH PHOSPHOLIPID BILAYERS" JOURNAL OF CHROMATOGRAPHY, vol. 597, no. 1/02, 24 April 1992 (1992-04-24), pages 293-297, XP000676280 ISSN: 0021-9673
- EPAND R M: "BIOPHYSICAL STUDIES OF LIPOPEPTIDE-MEMBRANE INTERACTIONS" BIOPOLYMERS, vol. 43, no. 1, 1 January 1997 (1997-01-01), pages 15-24, XP000677643 ISSN: 0006-3525
- MALETINSKA, LENKA ET AL: "Angiotensin analogues palmitoylated in positions 1 and 4" J.MED.CHEM., 1997, VOL. 43, NO. 20, PAGE(S) 3271-3279, XP002116343 US
- MALETINSKA, LENKA ET AL: "168. Lipid masking and reactivation of angiotensin analogues" HELV.CHIM.ACTA, 1996, VOL. 79, NO. 7, PAGE(S) 2023-2034, XP002116344 CH
- ISKANDRIAN AS ET AL: "Pharmacologic stress testing: mechanism of action, hemodynamic responses, and results in detection of coronary artery disease" JOURNAL OF MUCLEAR CARDIOLOGY, vol. 1, no. 1, January 1994 (1994-01), pages 94-111, XP002108106 ISSN: 1071-3581
- SHEHATA AR ET AL: "Direct comparison of arbutamine and dobutamine stress testing with myocardial perfusion imaging and echocardiography in patients with coronary artery disease" AMERICAN JOURNAL OF CARDIOLOGY, vol. 80, no. 6, 15 September 1997 (1997-09-15), pages 716-720, XP002108107
- PORTER T R ET AL: "THE EFFECT OF MICROBUBBLE GAS COMPOSITION AND EXTERNAL ULTRASOUND FREQUENCY ON THE NON-INVASIVE ENHANCEMENT OF ANTISENSE OLIGONUCLEOTIDE DELIVERY TO THE VASCULAR WALL IN PIGS" CIRCULATION, vol. 96, no. 8, 21 October 1997 (1997-10-21), page L-401 XP000198974 ISSN: 0009-7322

## Description

This invention relates to ultrasound imaging, more particularly to novel contrast agent preparations and their use in ultrasound imaging, for example in visualising tissue perfusion.

It is well known that contrast agents comprising dispersions of microbubbles of gases are particularly efficient backscatterers of ultrasound by virtue of the low density and ease of compressibility of the microbubbles. Such microbubble dispersions, if appropriately stabilised, may permit highly effective ultrasound visualisation of, for example, the vascular system and tissue microvasculature, often at advantageously low doses.

The use of ultrasonography to measure blood perfusion (i.e. blood flow per unit of tissue mass) is of potential value in, for example, tumour detection, tumour tissue typically having different vascularity from healthy tissue, and studies of the myocardium, e.g. to detect myocardial infarctions. A problem with the application of existing ultrasound contrast agents to cardiac perfusion studies is that the information content of images obtained is degraded by attenuation caused by contrast agent present in the ventricles of the heart.

In our copending International Patent Publication No. WO-A-9817324, the contents of which are incorporated herein by reference, we have disclosed that ultrasonic visualisation of a subject, in particular of perfusion in the myocardium and other tissues, may be achieved and/or enhanced by means of gas-containing contrast agent preparations which promote controllable and temporary growth of the gas phase in vivo following administration. Such contrast agent preparations may be used to promote controllable and temporary retention of the gas phase, for example in the form of microbubbles, in tissue microvasculature, thereby enhancing the concentration of gas in such tissue and accordingly enhancing its echogenicity, e.g. relative to the blood pool.

Such use of gas as a deposited perfusion tracer differs markedly from existing proposals regarding intravenously administrable microbubble ultrasound contrast agents. Thus it is generally thought necessary to avoid microbubble growth since, if uncontrolled, this may lead to potentially hazardous tissue embolisation. Accordingly it may be necessary to limit the dose administered and/or to use gas mixtures with compositions selected so as to minimise bubble growth *in vivo* by inhibiting inward diffusion of blood gases into the microbubbles (see e.g. WO-A-9503835 and WO-A-9516467).

In accordance with WO-A-9817324, on the other hand, a composition comprising a dispersed gas phase is coadministered with a composition comprising at least one substance which has or is capable of generating a gas or vapour pressure in vivo sufficient to promote controllable growth of the said dispersed gas phase through inward diffusion thereto of molecules of gas or vapour derived from said substance, which for brevity is hereinafter referred to as a "diffusible component", although it will be appreciated that transport mechanisms other than diffusion may additionally or alternatively be involved in operation of the invention, as discussed in greater detail hereinafter.

This coadministration of a dispersed gas phase-containing composition and a composition comprising a diffusible component having an appropriate degree of volatility may be contrasted with previous proposals regarding administration of volatile substances alone, e.g. in the form of phase shift colloids as described in WO-A-9416739. Thus the contrast agent preparations of WO-A-9817324 permit control of factors such as the probability and/or rate of growth of the dispersed gas by selection of appropriate constituents of the coadministered compositions, whereas administration of the aforementioned phase shift colloids alone may lead to generation of microbubbles which grow uncontrollably and unevenly, possibly to the extent where at least a proportion of the microbubbles may cause potentially dangerous embolisation of, for example, the myocardial vasculature and brain (see e.g. Schwarz, Advances in Echo-Contrast [1994(3)], pp. 48-49).

It has been found that administration of phase shift colloids alone may not lead to reliable or consistent *in vivo* volatilisation of the dispersed phase to generate gas or vapour microbubbles. Grayburn et al. in J. Am. Coll. Cardiol. 26(5) [1995], pp. 1340-1347 suggest that preactivation of perfluoropentane emulsions may be required to achieve myocardial opacification in dogs at effective imaging doses low enough to avoid haemodynamic side effects. An activation technique for such colloidal dispersions, involving application of hypobaric forces thereto, is described in WO-A-9640282; typically this involves partially filling a syringe with the emulsion and subsequently forcibly withdrawing and then releasing the plunger of the syringe to generate a transient pressure change which causes formation of gas microbubbles within the emulsion. This is an inherently somewhat cumbersome technique which may fail to give consistent levels of activation.

Again with regard to phase shift colloids, it is stated in US-A-5536489 that emulsions of water-insoluble gas-forming chemicals such as perfluoropentane may be used as contrast agents for site-specific imaging, the emulsions only generating a significant number of image-enhancing gas microbubbles upon application of ultrasonic energy to a specific location in the body which it is desired to image. Our own research has shown, however, that emulsions of volatile compounds such as 2-methylbutane or perfluoropentane give no detectable echo enhancement either *in vitro* or *in vivo* when ultrasonicated at energy levels which are sufficient to give pronounced contrast effects using two component contrast agents in accordance with WO-A-9817324.

The combined preparations of WO-A-9817324 are intended for simultaneous, separate or sequential use as a contrast agent in ultrasound imaging, and comprise:
i) an injectable aqueous composition having gas dispersed therein; and
ii) a composition comprising a diffusible component capable of diffusion *in vivo* into said dispersed gas so as at least transiently to increase the size thereof.

The preparations may advantageously be employed in visualising tissue perfusion in a subject, the increase in size of the dispersed gas being utilised to effect enrichment or temporary retention of gas in the microvasculature of such tissue, thereby enhancing its echogenicity.

A particular advantage of the preparations is that growth of the dispersed gas may be induced or enhanced by ultrasonication, by application of appropriate amounts of other forms of energy, including sound energy at lower or higher frequencies than those normally used in medical ultrasound imaging, shaking, vibration, an electric field or radiation, or by particle bombardment, for example with neutral particles, ions or electrons. This permits particularly effective control of factors such as the onset and rate of growth of the dispersed gas, and permits such growth to be localised to particular areas of the body of a subject, for example so as to effect temporary retention of gas in the microvasculature of a target organ, e.g. in the myocardium.

The present invention is based on the finding that the efficacy of contrast agent preparations of the type disclosed in WO-A-9817324 may be substantially enhanced if the two compositions are formulated in such a way that the dispersed gas component and diffusible component have affinity for each other, for example as a result of attractive electrostatic or other physical forces or of chemical (including biological) binding. This may be achieved by formulating the dispersed gas component as a stabilised gas dispersion and the diffusible component as a stabilised emulsion such that material present at the surfaces of the dispersed gas has affinity for material present at the surfaces of the dispersed diffusible component. The surface materials having affinity for each other may, for example, be materials such as surfactants which serve to stabilise the gas and diffusible component dispersions. Alternatively, surface materials with appropriate mutual affinity may be mixed with, chemically linked to or otherwise associated with non-affinity stabilising materials in the respective dispersions.

Whilst we do not wish to be bound by theoretical considerations, it is believed that the resulting affinity between the dispersed gas and the diffusible component increases the probability of interaction between them, e.g. by a factor of 10-100 times or even higher, so that a greater number of dispersed gas moieties are caused to grow for a given dose of the two components compared to the situation where the components lack such mutual affinity. This may particularly be the case where ultrasound or like activation is employed to induce growth of the dispersed gas. Here, in situations where there is no significant affinity between the components, it is thought that ultrasonication may lead to disintegration of a substantial proportion of the dispersed gas phase and only a relatively low level of interaction with diffusible component. The level of interaction may, however, be markedly increased by use of gas and diffusible components with mutual affinity.

Contrast agent preparations according to the invention may therefore be used at significantly lower doses than are suggested in WO-A-9817324 whilst giving equivalent contrast effects. This has valuable implications as regards product safety, since it may permit the use of diffusible component emulsions at such low levels that any risk of embolisation from the volatile content thereof, e.g. as described in J. Appl. Physiol. 40(5) [1976], pp. 745-751, is negligible, even after dilution with blood gases.

Alternatively or additionally, the dose of the gas dispersion may be reduced, with possible benefits as regards product safety and toxicity considerations. Such dose reduction may also lengthen the available imaging time window in applications such as echocardiography, by allowing earlier clearance of dispersed gas from ventricular blood and thereby permitting more rapid visualisation of gas retained in, for example, myocardial tissue.

Furthermore, it has been found that contrast agent preparations according to the invention may readily permit effective imaging of tissue such as the myocardium using conventional B-mode scanning techniques. Thus the ultrasound energy emitted by scanners operating in B-mode is sufficient to induce growth of the dispersed gas phase, which is then retained in the microvasculature and may be capable of generating diagnostically useful information for at least 5-10 minutes without undergoing ultrasound-induced deterioration. Such behaviour is markedly different from that exhibited by existing gas-containing contrast agents, which in general undergo relatively rapid degradation during ultrasonication and may therefore require use of more complex techniques, for example involving intermittent imaging, to effect satisfactory visualisation.

According to one aspect thereof, the present invention provides a combined preparation for simultaneous, separate or sequential use as a contrast agent in ultrasound imaging, said preparation comprising:
i) a first composition which is an injectable aqueous medium comprising dispersed gas and material serving to stabilise said gas; and
ii) a second composition which is an injectable oil-in-water emulsion wherein the oil phase comprises a diffusible component capable of diffusion in vivo into said dispersed gas so as at least transiently to increase the size thereof, said composition further comprising material serving to stabilise said emulsion,
characterised in that material present at the surfaces of the dispersed gas phase and material present at the surfaces of the dispersed oil phase have affinity for each other.

The invention further provides a method of generating enhanced images of a human or non-human animal subject which comprises the steps of:
i) injecting a first composition as defined above into the vascular system of said subject;
ii) before, during or after injection of said first composition injecting a second composition as defined above into said subject; and
iii) generating an ultrasound image of at least a part of said object.

The necessary affinity between surface materials respectively present in the first and second compositions may, for example, be achieved by using materials with opposite charges so that they interact and bind electrostatically to each other. Thus, for example, one of the surface materials may be a cationic surfactant and the other an anionic surfactant, e.g. as discussed in greater detail hereinafter. Charge differences between surface materials may also be achieved by incorporating appropriate cationic and/or anionic additives as necessary into stabilising materials, e.g. surfactants, present at the surfaces of either or both of the respective dispersed phases of the two compositions. Alternatively, the respective surface materials may comprise stabilisers or additives containing specific groups, molecules, ligands or vectors capable of interaction through chemical binding interactions such as covalent bonding, hydrogen bonding or ionic bonding. Thus the surface materials may, for example, respectively comprise an antigen and an antibody or fragment thereof, a lectin and a carbohydrate-containing group, avidin/streptavidin and biotin or a biotinyl group, a drug and a receptor, a transmitter and a receptor, a hormone and a receptor, a peptide or protein and a complementary peptide or protein, an enzyme or inactive enzyme and a substrate analogue or inhibitor, a nucleic acid (DNA or RNA) sequence and a complementary nucleic acid sequence, or a chelator and a ligand; the foregoing list is not to be considered limiting.

In general any biocompatible gas may be present in the gas dispersion used as the first composition in accordance with the invention, the term "gas" as used herein including any substances (including mixtures) at least partially, e.g. substantially or completely, in gaseous or vapour form at the normal human body temperature of 37°C. Representative gases thus include air; nitrogen; oxygen; carbon dioxide; hydrogen; inert gases such as helium, argon, xenon or krypton; sulphur fluorides such as sulphur hexafluoride, disulphur decafluoride or trifluoromethylsulphur pentafluoride; selenium hexafluoride; optionally halogenated silanes such as methylsilane or dimethylsilane; low molecular weight hydrocarbons (e.g. containing up to 7 carbon atoms), for example alkanes such as methane, ethane, a propane, a butane or a pentane, cycloalkanes such as cyclopropane, cyclobutane or cyclopentane, alkenes such as ethylene, propene, propadiene or a butene, or alkynes such as acetylene or propyne; ethers such as dimethyl ether; ketones; esters; halogenated low molecular weight hydrocarbons (e.g. containing up to 7 carbon atoms); or mixtures of any of the foregoing. Advantageously at least some of the halogen atoms in halogenated gases are fluorine atoms; thus biocompatible halogenated hydrocarbon gases may, for example, be selected from bromochlorodifluoromethane, chlorodifluoromethane, dichlorodifluoromethane, bromotrifluoromethane, chlorotrifluoromethane, chloropentafluoroethane, dichlorotetrafluoroethane, chlorotrifluoroethylene, fluoroethylene, ethylfluoride, 1,1-difluoroethane and perfluorocarbons. Representative perfluorocarbons include perfluoroalkanes such as perfluoromethane, perfluoroethane, perfluoropropanes, perfluorobutanes (e.g. perfluoro-n-butane, optionally in admixture with other isomers such as perfluoro-iso-butane), perfluoropentanes, perfluorohexanes or perfluoroheptanes; perfluoroalkenes such as perfluoropropene, perfluorobutenes (e.g. perfluorobut-2-ene), perfluorobutadiene, perfluoropentenes (e.g. perfluoropent-1-ene) or perfluoro-4-methylpent-2-ene; perfluoroalkynes such as perfluorobut-2-yne; and perfluorocycloalkanes such as perfluorocyclobutane, perfluoromethylcyclobutane, perfluorodimethylcyclobutanes, perfluorotrimethylcyclobutanes, perfluorocyclopentane, perfluoromethyl-cyclopentane, perfluorodimethylcyclopentanes, perfluorocyclohexane, perfluoromethylcyclohexane or perfluorocycloheptane. Other halogenated gases include methyl chloride, fluorinated (e.g. perfluorinated) ketones such as perfluoroacetone and fluorinated (e.g. perfluorinated) ethers such as perfluorodiethyl ether. The use of perfluorinated gases, for example sulphur hexafluoride and perfluorocarbons such as perfluoropropane, perfluorobutanes, perfluoropentanes and perfluorohexanes, may be particularly advantageous in view of the recognised high stability in the bloodstream of microbubbles containing such gases. Other gases with physicochemical characteristics which cause them to form highly stable microbubbles in the bloodstream may likewise be useful.

The gas may, for example, be present in the first composition in the form of microbubbles at least partially encapsulated or otherwise stabilised by gas-stabilising material. This stabilising material may, for example, comprise a coalescence-resistant surface membrane (for example gelatin, e.g. as described in WO-A-8002365), a filmogenic protein (for example an albumin such as human serum albumin, e.g. as described in US-A-4718433, US-A-4774958, US-A-4844882, EP-A-0359246, WO-A-9112823, WO-A-9205806, WO-A-9217213, WO-A-9406477 or WO-A-9501187), a polymer material (for example a synthetic biodegradable polymer as described in EP-A-0398935, an elastic interfacial synthetic polymer membrane as described in EP-A-0458745, a microparticulate biodegradable polyaldehyde as described in EP-A-0441468, a microparticulate N-dicarboxylic acid derivative of a polyamino acid - polycyclic imide as described in EP-A-0458079, or a biodegradable polymer as described in WO-A-9317718 or WO-A-9607434), a non-polymeric and non-polymerisable wall-forming material (for example as described in WO-A-9521631), or a surfactant (for example a polyoxyethylene-polyoxypropylene block copolymer surfactant such as a Pluronic, a polymer surfactant as described in WO-A-9506518, or a film-forming surfactant such as a phospholipid, e.g. as described in WO-A-9211873, WO-A-9217212, WO-A-9222247, WO-A-9428780, WO-A-9503835 or WO-A-9729783).

The first composition may also be derived from gas-containing solid systems, for example microparticles (especially aggregates of microparticles) having gas contained therewithin or otherwise associated therewith (for example being adsorbed on the surface thereof and/or contained within voids, cavities or pores therein, e.g. as described in EP-A-0122624, EP-A-0123235, EP-A-0365467, WO-A-9221382, WO-A-9300930, WO-A-9313802, WO-A-9313808 or WO-A-9313809). It will be appreciated that the echogenicity of such microparticulate contrast agents may derive directly from the contained/associated gas and/or from gas (e.g. microbubbles) liberated from the solid material (e.g. upon dissolution of the microparticulate structure).

The disclosures of all of the above-described documents relating to gas-containing formulations are incorporated herein by reference.

Gas microbubbles and other gas-containing materials such as microparticles preferably have an initial average size not exceeding 10 µm (e.g. of 7 µm or less) in order to permit their free passage through the pulmonary system following administration, e.g. by intravenous injection. However, larger microbubbles may be employed where, for example, these contain a mixture of one or more relatively blood-soluble or otherwise diffusible gases such as air, oxygen, nitrogen or carbon dioxide with one or more substantially insoluble and non-diffusible gases such as perfluorocarbons. Outward diffusion of the soluble/diffusible gas content following administration will cause such microbubbles rapidly to shrink to a size which will be determined by the amount of insoluble/non-diffusible gas present and which may be selected to permit passage of the resulting microbubbles through the lung capillaries of the pulmonary system.

Since dispersed gas administered in accordance with the invention is caused to grow *in vivo* through interaction with diffusible component, the minimum size of the microbubbles, solid-associated gas etc. as administered may be substantially lower than the size normally thought necessary to provide significant interaction with ultrasound (typically ca. 1-5 µm at conventionally-employed imaging frequencies); the dispersed gas moieties may therefore have sizes as low as, for example, 1 nm or below. The invention may accordingly permit use of gas-containing compositions which have not hitherto been proposed for use as ultrasound contrast agents, e.g. because of the low size of the dispersed gas moieties.

Where phospholipid-containing first compositions are employed in accordance with the invention, e.g. in the form of phospholipid-stabilised gas microbubbles, representative examples of useful phospholipids include lecithins (i.e. phosphatidylcholines), for example natural lecithins such as egg yolk lecithin or soya bean lecithin, semisynthetic (e.g. partially or fully hydrogenated) lecithins and synthetic lecithins such as dimyristoylphosphatidylcholine, dipalmitoylphosphatidylcholine or distearoylphosphatidylcholine; phosphatidic acids; phosphatidylethanolamines; phosphatidylserines; phosphatidylglycerols; phosphatidylinositols; cardiolipins; sphingomyelins; fluorinated analogues of any of the foregoing; mixtures of any of the foregoing and mixtures with other lipids such as cholesterol. The use of phospholipids predominantly (e.g. at least 75%) comprising molecules individually bearing net overall charge, e.g. negative charge, for example as in naturally occurring (e.g. soya bean or egg yolk derived), semisynthetic (e.g. partially or fully hydrogenated) and synthetic phosphatidylserines, phosphatidylglycerols, phosphatidylinositols, phosphatidic acids and/or cardiolipins, for example as described in WO-A-9729783, may be particularly advantageous.

Representative examples of gas-containing microparticulate materials which may be useful in first compositions in accordance with the invention include carbohydrates (for example hexoses such as glucose, fructose or galactose; disaccharides such as sucrose, lactose or maltose; pentoses such as arabinose, xylose or ribose; α-, β- and γ-cyclodextrins; polysaccharides such as starch, hydroxyethyl starch, amylose, amylopectin, glycogen, inulin, pulullan, dextran, carboxymethyl dextran, dextran phosphate, ketodextran, aminoethyldextran, alginates, chitin, chitosan, hyaluronic acid or heparin; and sugar alcohols, including alditols such as mannitol or sorbitol), inorganic salts (e.g. sodium chloride), organic salts (e.g. sodium citrate, sodium acetate or sodium tartrate), X-ray contrast agents (e.g. any of the commercially available carboxylic acid and non-ionic amide contrast agents typically containing at least one 2,4,6-triiodophenyl group having substituents such as carboxyl, carbamoyl, N-alkylcarbamoyl, N-hydroxyalkylcarbamoyl, acylamino, N-alkylacylamino or acylaminomethyl at the 3- and/or 5-positions, as in metrizoic acid, diatrizoic acid, iothalamic acid, ioxaglic acid, iohexol, iopentol, iopamidol, iodixanol, iopromide, metrizamide, iodipamide, meglumine iodipamide, meglumine acetrizoate and meglumine diatrizoate), and polypeptides and proteins (e.g. gelatin or albumin such as human serum albumin).

Other gas-containing materials which may be useful in first compositions in accordance with the invention include gas-containing material stabilised by metals (e.g. as described in US-A-3674461 or US-A-3528809), gas-containing material stabilised by synthetic polymers (e.g. as described in US-A-3975194 or by Farnand in Powder Technology 22 [1979], pp. 11-16), commercially available microspheres of the Expancel® type, e.g. Expancel 551 DE (see e.g. Eur. Plast. News 9(5) [1982], p. 39, Nonwovens Industry [1981], p. 21 and Mat. Plast. Elast. 10 [1980], p. 468), commercially available microspheres of the Ropaque® type (see e.g. J. Coatings Technol. 55(707) [1983], p. 79), micro- and nano-sized gas-containing structures such as zeolites, inorganic or organic aerogels, nanosized open void-containing chemical structures such as fullerenes, clathrates or nanotubes (e.g. as described by G.E. Gadd in Science 277 (5328) [1997], pp. 933-936), and natural surfactant-stabilised microbubble dispersions (e.g. as described by d'Arrigo in "Stable Gas-in-Liquid Emulsions, Studies in physical and theoretical chemistry" 40 - Elsevier, Amsterdam [1986]).

The dispersed oil phase in the second composition of preparations according to the invention may comprise any appropriate diffusible component which is at least partially insoluble in and immiscible with water. The diffusible component in such emulsions is advantageously a liquid at processing and storage temperature, which may for example be as low as -10°C if the aqueous phase contains appropriate antifreeze material, while being a gas or exhibiting a substantial vapour pressure at body temperature. Appropriate compounds may, for example, be selected from the various lists of emulsifiable low boiling liquids given in the aforementioned WO-A-9416379, the contents of which are incorporated herein by reference. Specific examples of emulsifiable diffusible components include aliphatic ethers such as diethyl ether; polycyclic oils or alcohols such as menthol, camphor or eucalyptol; heterocyclic compounds such as furan or dioxane; aliphatic hydrocarbons, which may be saturated or unsaturated and straight chained or branched, e.g. as in n-butane, n-pentane, 2-methylpropane, 2-methylbutane, 2,2-dimethylpropane, 2,2-dimethylbutane, 2,3-dimethylbutane, 1-butene, 2-butene, 2-methylpropene, 1,2-butadiene, 1,3-butadiene, 2-methyl-1-butene, 2-methyl-2-butene, isoprene, 1-pentene, 1,3-pentadiene, 1,4-pentadiene, butenyne, 1-butyne, 2-butyne or 1,3-butadiyne; cycloaliphatic hydrocarbons such as cyclobutane, cyclobutene, methylcyclopropane or cyclopentane; and halogenated low molecular weight hydrocarbons (e.g. containing up to 7 carbon atoms). Representative halogenated hydrocarbons include dichloromethane, methyl bromide, 1,2-dichloroethylene, 1,1-dichloroethane, 1-bromoethylene, 1-chloroethylene, ethyl bromide, ethyl chloride, 1-chloropropene, 3-chloropropene, 1-chloropropane, 2-chloropropane and t-butyl chloride. Advantageously at least some of the halogen atoms are fluorine atoms, for example as in dichlorofluoromethane, trichlorofluoromethane, 1,2-dichloro-1,2-difluoroethane, 1,2-dichloro-1,1,2,2-tetrafluoroethane, 1,1,2-trichloro-1,2,2-trifluoroethane, 2-bromo-2-chloro-1,1,1-trifluoroethane, 2-chloro-1,1,2-trifluoroethyl difluoromethyl ether, 1-chloro-2,2,2-trifluoroethyl difluoromethyl ether, partially fluorinated alkanes (e.g. pentafluoropropanes such as 1H,1H,3H-pentafluoropropane, hexafluorobutanes, nonafluorobutanes such as 2H-nonafluoro-t-butane, decafluoropentanes such as 2H,3H-decafluoropentane, and tridecafluorohexanes such as 1H-tridecafluorohexane), partially fluorinated alkenes (e.g. heptafluoropentenes such as 1H,1H,2H-heptafluoropent-1-ene, and nonafluorohexenes such as 1H,1H,2H-nonafluorohex-1-ene), fluorinated ethers (e.g. 1,1,2,2-tetrafluoroethyl methyl ether, 2,2,3,3,3-pentafluoropropyl methyl ether, 1,1,2,3,3,3-hexafluoropropyl methyl ether or 2,2,3,3,3-pentafluoropropyl difluoromethyl ether) and, more preferably, perfluorocarbons. Examples of perfluorocarbons include perfluoroalkanes such as perfluorobutanes, perfluoropentanes, perfluorohexanes (e.g. perfluoro-2-methylpentane), perfluoroheptanes, perfluorooctanes, perfluorononanes and perfluorodecanes; perfluorocycloalkanes such as perfluorocyclobutane, perfluorodimethylcyclobutanes, perfluorocyclopentane and perfluoromethylcyclopentane; perfluoroalkenes such as perfluorobutenes (e.g. perfluorobut-2-ene or perfluorobuta-1,3-diene), perfluoropentenes (e.g. perfluoropent-1-ene) and perfluorohexenes (e.g. perfluoro-2-methylpent-2-ene or perfluoro-4-methylpent-2-ene); perfluorocycloalkenes such as perfluorocyclopentene or perfluorocyclopentadiene; and perfluorinated alcohols such as perfluoro-t-butanol.

If desired, the diffusible component may be formulated as part of a proprietary registered pharmaceutical emulsion, such as Intralipid® (Pharmacia).

In a further embodiment of the invention, the oil phase may be a mixture of two fluids, the first being, for example, a perfluorocarbon as discussed above, e.g. perfluorodimethylcyclobutane, and the other being a volatile lipophilic "filling" substance having somewhat higher water solubility, for example a halogenated inhalation anaesthetic or a hydrocarbon. The purpose of the "filling" substance is to cause a non-specific increase in microbubble size. Following initiation of growth of the dispersed gas phase, microbubbles will rapidly shrink after their initial growth as a result of loss of the "filling" substance by outward diffusion. The remaining microbubbles, now containing only the first volatile compound of lower water solubility and blood gases, will have a reduced size which can be controlled by appropriate selection of the initial ratio of the two volatile fluids in the diffusible component emulsion. A representative mixing ratio for the two fluids may be 1:9 perfluorocarbon:"filling" substance.

The emulsion-stabilising material may typically comprise one or more surfactants. It will be appreciated that the nature of such surfactants may significantly affect factors such as the rate of growth of the dispersed gas phase. In general a wide range of surfactants may be useful, for example selected from the extensive lists given in EP-A-0727225, the contents of which are incorporated herein by reference. Representative examples of useful surfactants include fatty acids (e.g. straight chain saturated or unsaturated fatty acids, for example containing 10-20 carbon atoms) and carbohydrate and triglyceride esters thereof, phospholipids (e.g. lecithin), fluorine-containing phospholipids, proteins (e.g. albumins such as human serum albumin), polyethylene glycols, block copolymer surfactants (e.g. polyoxyethylene-polyoxypropylene block copolymers such as Pluronics, extended polymers such as acyloxyacyl polyethylene glycols, for example polyethyleneglycol methyl ether 16-hexadecanoyloxy-hexadecanoate, e.g. wherein the polyethylene glycol moiety has a molecular weight of 2300, 5000 or 10000), fluorine-containing surfactants (e.g. as marketed under the trade names Zonyl and Fluorad, or as described in WO-A-9639197, the contents of which are incorporated herein by reference), and cationic surfactants, for example comprising one or more quaternary ammonium groups and one or more lipid groups such as long chain (e.g. C₁₀₋₃₀) alkyl or alkanoyl groups.

The use of cationic substances, e.g. as surfactants or other stabilisers or as additives to stabilisers, in surface material present in diffusible component emulsions in accordance with the invention may be particularly advantageous in conjunction with gas dispersions containing anionic surface materials, for example negatively charged phospholipids such as naturally occuring (e.g. soya bean- or egg yolk-derived), semisynthetic (e.g. partially or fully hydrogenated) or synthetic phospholipids such as phosphatidylserines, phosphatidylglycerols, phosphatidylinositols, phosphatidic acids and cardiolipins, in view of the consequent electrostatic interaction between the two surface materials.

In general a wide range of cationic substances may be used, for example at least somewhat hydrophobic and/or substantially water-insoluble compounds having a basic nitrogen atom, e.g. as in primary amines, secondary amines, tertiary amines and alkaloids, including pyrrolidines, piperidines, imidazoles, pyridines, quinolines and alkyl- and aryl-guanidinium compounds. Examples of representative cationic substances include lipophilic quaternary ammonium or pyridinium salts such as didodecyldimethylammonium bromide, cetyltrimethyl-ammonium chloride, cetylpyridinium chloride, cetyltrimethylammonium bromide, Quaternium-26, oleyltrimethylammonium chloride, cetylethyldimethyl-ammonium bromide, lapyrium chloride, Halimide®, cetalkonium chloride, 1,2-distearoyl-3-trimethyl-ammoniumpropane, betaine cetyl ester or DC-cholesterol; lipophilic secondary or tertiary amines such as diethyl-stearylamine, methylstearylamine, dimethylsphingosine, esters of fatty alcohols with dimethylglycine, esters of fatty acids with dimethylethanolamine, esters of fatty alcohols with sarcosine, or esters of fatty alcohols with N(2)- or N(6)-dimethyllysine; amides of fatty acids with substituted di- or tri-amines, such as N-stearoyl-N'-dimethylaminopropylamine; primary amines such as stearylamine or dodecylamine; esters of fatty alcohols with amino acids such as alanine, lysine, serine or threonine, as in alanine cetyl ester or lysine cetyl ester; amides of fatty acids with di- or tri-amines, such as monostearoyldiaminopropane or monostearoylputrescine; or positively charged phospholipids such as dialkyl-sn-glyceroethylphosphatidylcholines or esters of phosphatidic acids such as dipalmitoylphosphatidic acid or distearoylphosphatidic acid with aminoalcohols such as lysine hydroxyethylamide, hydroxylysine ethyl ester, 1,3-diamino-2-propanol or 2,4-diaminobenzyl alcohol. Lipophilic cationic compounds comprising a positively charged atom other than nitrogen, for example sulphur (e.g. as in sulphonium compounds), iodine (e.g. as in iodonium compounds), selenium or phosphorus (e.g. as in phosphonium compounds), as well as appropriate positively charged metal complexes, may also be useful.

Preferred cationic substances include compounds which are either endogenous (for example sphingosine, DL-dihydrosphingosine, dimethylsphingosine, phytosphingosine or psycosine) or are readily degradable into endogenic substances (for example esters or amides of choline, ethanolamine, putrescine, lysine, arginine, glycine, sarcosine, dimethylglycine, carnitine, betaine or spermidine, e.g. as in cetyl betaine ester, or derivatives of amino acids in general). The use of fluorine-containing cationic surfactants, e.g. fluorinated positively charged phospholipids or fluorinated cationic surfactants as marketed under the trade name Zonyl, may also be advantageous.

The second composition may, for example, be injected intravenously, intramuscularly or subcutaneously; the latter routes may be advantageous where it is desired specifically to limit the effect of the diffusible component to a particular target area of a subject. One example of a composition for subcutaneous injection comprises nanoparticles such as are used for lymph angiography.

The droplet size of emulsions intended for intravenous injection should preferably be less than 10 µm, e.g. less than 7 µm, and greater than 0.1 µm in order to facilitate unimpeded passage through the pulmonary system. It may be advantageous to employ first and second compositions respectively comprising dispersed gas microbubbles and dispersed diffusible droplets which have substantially similar sizes, for example having diameters in the range 1-7, e.g. 2-6 µm.

If desired, the diffusible component may also be formulated as a microemulsion. Such systems are advantageous by virtue of their thermodynamic stability and the fact that the diffusible component is in practice uniformly distributed throughout the aqueous phase; microemulsions therefore have the appearance of solutions but may exhibit the properties of emulsions as regards the partial pressure of the dispersed phase.

As noted above, the invention permits the use of substantially lower doses of diffusible component-containing emulsion than has hitherto been thought necessary. Phase shift colloid contrast agents such as are disclosed in WO-A-9416739 are typically administered in amounts corresponding to ca. 0.1 ml dispersed phase/kg body weight. It is stated in WO-A-9817324 that where the diffusible component is a perfluorocarbon formulated as an oil-in-water emulsion this may typically be administered at a dose corresponding to 0.2-1.0 µl perfluorocarbon/kg body weight. The present invention, however, permits images comparable to those observed in WO-A-9817324 to be obtained using at least 20-fold and possibly up to 200-fold lower doses of diffusible component, for example in the range 1-100 nl diffusible component/kg body weight, e.g. 20 nl diffusible component/kg body weight.

Whilst the diffusible component content of emulsions has the capability for at least a 100-fold increase in volume when evaporated, it will be appreciated that at such doses the total administered amount of diffusible component will in general be insufficient to give rise to risks of embolism. Moreover, it is likely that such doses are below any threshold at which gas bubbles might spontaneously be generated in low-pressure venous compartments of the circulation (e.g. the vena cava, right heart chambers and pulmonary artery) as a result of the volatile diffusible component and blood gases supersaturating the blood.

In order to ensure maximum volatilisation of the diffusible component following administration and to enhance growth of the dispersed gas, both of which are endothermic processes, it may be advantageous to manipulate the temperature of the first and/or the second composition prior to administration and/or to incorporate exothermically reactive constituents therein; the use of such constituents which react exothermically under the influence of ultrasound radiation may be particularly advantageous.

Growth of the dispersed gas phase *in vivo* may, for example, be accompanied by expansion of any encapsulating stabilising material (where this has sufficient flexibility) and/or by abstraction of excess surfactant or other stabilising material, e.g. from the second composition, to the growing gas-liquid interfaces. It is also possible, however, that stretching of the encapsulating material and/or interaction of the material with ultrasound may substantially increase its porosity. Whereas such disruption of encapsulating material has hitherto in many cases been found to lead to rapid loss of echogenicity through outward diffusion and dissolution of the gas thereby exposed, we have found that when using contrast agent preparations in accordance with the present invention, the exposed gas exhibits substantially stability. Whilst not wishing to be bound by theoretical calculations, we believe that the exposed gas, e.g. in the form of liberated microbubbles, may be stabilised, e.g. against collapse of the microbubbles, by the supersaturated environment generated by the diffusible component, which provides an inward pressure gradient to counteract the outward diffusive tendency of the microbubble gas. The exposed gas surface, by virtue of the substantial absence of encapsulating material, may cause the contrast agent preparation to exhibit exceptionally favourable acoustic properties as evidenced by high backscatter and low energy absorption (e.g. as expressed by high backscatter: attenuation ratios); this echogenic effect may continue for a significant period, even during continuing ultrasound irradiation.

The stabilising effect of coadministered diffusible component may therefore be used to great advantage to enhance both the duration and magnitude of the echogenicity of existing gas-containing contrast agent formulations in cases where these parameters may be insufficient when the contrast agent composition is administered alone. Thus, for example, the duration of effect of albumin-based contrast agents is often severely limited by collapse of the encapsulating albumin material, either as a result of systolic pressure changes in the heart or venous system or as a consequence of ultrasound irradiation, but may be substantially enhanced by coadministration with a diffusible component in accordance with the present invention.

In a representative embodiment of the method of the invention a composition comprising a dispersed gas component and a composition comprising an emulsified diffusible component are selected such that, following intravenous injection of the two compositions, at least a proportion of the dispersed gas passes through the lungs and then undergoes rapid growth following passage from the lungs through inward diffusion of the diffusible component, so as temporarily to be retained in the myocardium and thereby permit ultrasonic visualisation of myocardial perfusion. As the concentration of volatile diffusible component in the bloodstream falls away, e.g. as the component is cleared from the blood, for example by removal through the lungs and exhalation by the subject, by metabolism or by redistribution to other tissues, the diffusible component will typically diffuse out of the dispersed gas, which will therefore shrink towards its initial smaller size, and ultimately once more becoming free flowing in the bloodstream, typically being removed therefrom by the reticuloendothelial system. This pattern of a substantial transient increase in echogenicity followed by disappearance of contrast effect is markedly different from any echogenic properties exhibited by either of the two compositions when administered alone. It will be appreciated from the foregoing that control of the duration of retention of the dispersed gas may therefore be achieved by appropriate adjustment of the dose and/or formulation of the diffusible component, particularly of the nature and degree of affinity between the gas component and diffusible component.

Other capillary systems, such as but not limited to those of the kidney, liver, spleen, thyroid, skeletal muscle, breast and prostate, may similarly be imaged.

In general, the rate and/or extent of growth of the dispersed gas may be controlled by appropriate selection of the gas and the gas-stabilising material and, more particularly, the nature of the emulsified diffusible component and the manner in which it is formulated, including the nature of the emulsion-stabilising material and the size of the emulsion droplets. In this last context, for a given amount of emulsified diffusible component, a reduction in droplet size may enhance the rate of transfer of diffusible component relative to that from larger droplets since more rapid release may occur from smaller droplets having higher surface area:volume ratios. Other parameters permitting control include the relative amounts in which the two compositions are administered and, where these are administered separately, the order of administration, the time interval between the two administrations, and possible spatial separation of the two administrations. In this last respect it will be appreciated that the inherent diffusivity of the diffusible component may permit its application to different parts of the body in a wide variety of ways, for example subcutaneously, intravenously or intramuscularly.

Particularly important parameters with regard to the diffusible component are its solubility in water/blood and its diffusibility (e.g. as expressed by its diffusion constants), which will determine its rate of transport through the carrier liquid or blood, and its permeability through any membrane of stabilising material encapsulating the dispersed gas. The pressure generated by the diffusible component *in vivo* will also affects its rate of diffusion into the dispersed gas, as will its concentration. Thus, in accordance with Fick's law, the concentration gradient of diffusible component relative to the distance between, for example, individual gas microbubbles and emulsion droplets, together with the diffusion coefficient of the diffusible substance in the surrounding liquid medium, will determine the rate of transfer by simple diffusion; the concentration gradient is determined by the solubility of the diffusible component in the surrounding medium and the distance between individual gas microbubbles and emulsion droplets. Similarly, the water solubility, vapour pressure and molecular size of the diffusible component will affect the lifetime of expanded microbubbles by the influence of these parameters on the diffusion rate of the diffusible component. This accordingly permits control of contrast duration, which optimally may be between 2 and 5 minutes.

The effective rate of transport of the diffusible component may, if desired, be controlled by adjusting the viscosity of the dispersed gas phase composition and/or the diffusible component composition, for example by incorporating one or more biocompatible viscosity enhancers such as X-ray contrast agents, polyethylene glycols, carbohydrates, proteins, polymers or alcohols into the formulation. It may, for example, be advantageous to coinject the two compositions as a relatively high volume bolus (e.g. having a volume of at least 20 ml in the case of a 70 kg human subject), since this will delay complete mixing of the constituents with blood (and thus the onset of growth of the dispersed gas) until after entry into the right ventricle of the heart and the lung capillaries. The delay in growth of the dispersed gas may be maximised by employing carrier liquid which is undersaturated with respect to gases and any other diffusible components as hereinbefore defined, e.g. as a result of being cooled.

As noted above, transport mechanisms other than diffusion may be involved in operation of the invention. Thus, for example, transport may also occur through hydrodynamic flow within the surrounding liquid medium; this may be important in vessels and capillaries where high shear rate flow may occur. Transport of diffusible component to the dispersed gas may also occur as a result of collision or near-collision processes, e.g. between gas microbubbles and emulsion droplets, for example leading to adsorption of diffusible component at the microbubble surface and/or penetration of diffusible component into the microbubble, i.e. a form of coalescence. In such cases the diffusion coefficient and solubility of the diffusible component have a minimal effect on the rate of transfer, the particle size of the diffusible component (e.g. the droplet size where this is formulated as an emulsion) and the collision frequency between microbubbles and droplets being the principal factors controlling the rate and extent of microbubble growth. Thus, for example, for a given amount of emulsified diffusible component, a reduction in droplet size will lead to an increased overall number of droplets and so may enhance the rate of transfer by reducing the mean interparticle distance between the gas microbubbles and emulsion droplets and thus increasing the probability of collision and/or coalescence. As noted above, the rate of transfers proceeding through collision processes may be markedly enhanced if additional oscillatory movement is imparted to the gas microbubbles and emulsion droplets of the diffusible component through application of ultrasonic energy. The kinetics of collision processes induced by such ultrasonic energy may differ from the kinetics for transport of diffusible component in carrier liquid and/or blood, for example in that specific energy levels may be necessary to initiate coalescence of colliding gas microbubbles and emulsion droplets. Accordingly it may be advantageous to select the size and therefore the mass of the emulsion droplets so that they generate sufficient collisional force with the oscillating microbubbles to induce coalescence.

As also noted above, the permeability of any stabilising material encapsulating the dispersed gas phase is a parameter which may affect the rate of growth of the gas phase, and it may therefore be desirable to select a diffusible component which readily permeates any such encapsulating material (which may, for example, be a polymer or surfactant membrane, e.g. a monolayer or one or more bilayers of a membrane-forming surfactant such as a phospholipid). We have found, however, that substantially impermeable encapsulating material may also be used, since it appears that sonication- or other energy input-induced growth of the dispersed gas may occur despite the presence of such impermeable material.

Whilst we do not wish to be bound by theoretical considerations it may be that ultrasonication at least transiently modifies the permeability of the encapsulating material, the diffusibility of the diffusible component in the surrounding liquid phase and/or the frequency of collisions between emulsion droplets and the encapsulated microbubbles. Since the effect may be observed using extremely short ultrasound pulses (e.g. with durations of ca. 0.3 µs in B-mode imaging or ca. 2 µs in Doppler or second harmonic imaging) it seems unlikely to be an example of rectified diffusion, in which ongoing ultrasound irradiation produces a steady increase in the equilibrium radii of gas bubbles (see Leighton, E.G. - "The Acoustic Bubble", Academic Press [1994], p. 379), and it may be that the ultrasound pulses disrupt the encapsulating membrane and so enhance growth of the dispersed gas through inward diffusion of diffusible component into the thus-exposed gas phase.

If desired, either the dispersed gas or the diffusible component may comprise an azeotropic mixture or may be selected so that an azeotropic mixture is formed *in vivo* as the diffusible component mixes with the dispersed gas. Such azeotrope formation may, for example, be used effectively to enhance the volatility of relatively high molecular weight compounds, e.g. halogenated hydrocarbons such as fluorocarbons (including perfluorocarbons) which under standard conditions are liquid at the normal human body temperature of 37°C, such that they may be administered in gaseous form at this temperature. This has substantial benefits as regards the effective echogenic lifetime in vivo of contrast agents containing such azeotropic mixtures since it is known that parameters such as the water solubility, fat solubility, diffusibility and pressure resistivity of compounds such as fluorocarbons decrease with increasing molecular weight. Contrast agents containing biocompatible azeotropic mixtures which are gaseous at 37°C are described in WO-A-9847540, the contents of which are incorporated herein by reference.

In general, the recognised natural resistance of azeotropic mixtures to separation of their constituents will enhance the stability of contrast agent components containing the same, both during preparation, storage and handling and following administration.

Azeotropic mixtures useful in accordance with the invention may, for example, be selected by reference to literature relating to azeotropes, by experimental investigation and/or by theoretical predictions, e.g. as described by Tanaka in Fluid Phase Equilibria 24 (1985), pp. 187-203, by Kittel, C. and Kroemer, H. in Chapter 10 of Thermal Physics (W.H. Freeman & Co., New York, USA, 1980) or by Hemmer, P.C. in Chapters 16-22 of Statistisk Mekanikk (Tapir, Trondheim, Norway, 1970), the contents of which are incorporated herein by reference.

One literature example of an azeotrope which effectively reduces the boiling point of the higher molecular weight component to below normal body temperature is the 57:43 w/w mixture of 1,1,2-trichloro-1,2,2-trifluoromethane (b.p. 47.6°C) and 1,2-difluoromethane (b.p. 29.6°C) described in US-A-4055049 as having an azeotropic boiling point of 24.9°C. Other examples of halocarbon-containing azeotropic mixtures are disclosed in EP-A-0783017, US-A-5599783, US-A-5605647, US-A-5605882, US-A-5607616, US-A-5607912, US-A-5611210, US-A-5614565 and US-A-5616821, the contents of which are incorporated herein by reference.

Simons et al. in J. Chem. Phys. 18(3) (1950), pp. 335-346 report that mixtures of perfluoro-*n*-pentane (b.p. 29°C) and n-pentane (b.p. 36°C) exhibit a large positive deviation from Raoult's law; the effect is most pronounced for approximately equimolar mixtures. In practice the boiling point of the azeotropic mixture has been found to be about 22°C or less. Mixtures of perfluorocarbons and unsubstituted hydrocarbons may in general exhibit useful azeotropic properties; strong azeotropic effects have been observed for mixtures of such components having substantially similar boiling points. Examples of other perfluorocarbon:hydrocarbon azeotropes include mixtures of perfluoro-n-hexane (b.p. 59°C) and *n*-pentane, where the azeotrope has a boiling point between room temperature and 35°C, and of perfluoro-4-methylpent-2-ene (b.p. 49°C) and n-pentane, where the azeotrope has a boiling point of approximately 25°C.

Other potentially useful azeotropic mixtures include mixtures of halothane and diethyl ether and mixtures of two or more fluorinated gases, for example perfluoropropane and fluoroethane, perfluoropropane and 1,1,1-trifluoroethane, or perfluoroethane and difluoromethane.

It is known that fluorinated gases such as perfluoroethane may form azeotropes with carbon dioxide (see e.g. WO-A-9502652). Accordingly, administration of contrast agents containing such gases may lead to in vivo formation of ternary or higher azeotropes with blood gases such as carbon dioxide, thereby further enhancing the stability of the dispersed gas.

Where the two compositions of combined contrast agent preparations according to the invention are to be administered simultaneously they may, for example, be injected from separate syringes via suitable coupling means or may be premixed, preferably under controlled conditions such that premature growth of the dispersed gas is avoided.

Compositions intended for mixing prior to simultaneous administration may advantageously be stored in appropriate dual or multi-chamber devices. Thus, for example, the dispersed gas-containing first composition or a dried precursor therefor [e.g. comprising a lyophilised residue of a suspension of gas microbubbles in an amphiphilic material-containing aqueous medium, particularly wherein the amphiphilic material consists essentially of phospholipid predominantly (e.g. at least 75%, preferably substantially completely) comprising molecules which individually have an overall net (e.g. negative) charge] may be contained in a first chamber such as a vial, to which a syringe containing the diffusible component-containing second composition is sealing connected; the syringe outlet is closed, e.g. with a membrane or plug, to avoid premature mixing. Operation of the syringe plunger ruptures the membrane and causes the second composition to mix with the first composition or to mix with and reconstitute a precursor therefor; following any necessary or desired shaking and/or dilution, the mixture may be withdrawn (e.g. by syringe) and administered.

Alternatively the two compositions may be stored within a single sealed vial or syringe, being separated by, for example, a membrane or plug; an overpressure of gas or vapour may be applied to either or both compositions. Rupture of the membrane or plug, e.g. by insertion of a hypodermic needle into the vial, leads to mixing of the compositions; this may if desired be enhanced by hand-shaking, whereafter the mixture may be withdrawn and administered. Other embodiments, for example in which a vial containing a dried precursor for the first composition is fitted with a first syringe containing a redispersion fluid for said precursor and a second syringe containing the second composition, or in which a vial containing membrane-separated second composition and dried precursor for the first composition is fitted with a syringe containing redispersion fluid for the latter, may similarly be used.

In embodiments of the invention in which the two compositions are mixed prior to administration, either at the manufacturing stage or subsequently, the mixture will typically be stored at elevated pressure or reduced temperature such that the pressure of the diffusible component is insufficient to provide growth of the dispersed gas. Activation of growth of the dispersed gas may be induced simply by release of excess pressure or by the heating to body temperature which will follow administration of the mixture, or it may if desired be brought about by preheating the mixture immediately before administration.

In embodiments of the invention in which the two compositions are administered separately, the timing between the two administrations may be used to influence the area of the body in which growth of the dispersed gas phase predominantly occurs. Thus, for example, the second composition may be injected first and the diffusible component allowed to concentrate in the liver, thereby enhancing imaging of that organ upon subsequent injection of the dispersed gas-containing first composition. Where the stability of the gas dispersion permits, this may likewise be injected first and allowed to concentrate in the liver, with the diffusible component-containing second composition then being administered to enhance the echogenicity thereof.

Representative ultrasound imaging techniques which may be useful in accordance with the invention include fundamental B-mode imaging; harmonic B-mode imaging, including reception of sub-harmonics and the second or higher harmonics; tissue Doppler imaging, optionally including selective reception of fundamental, harmonic or sub-harmonic echo frequencies; colour Doppler imaging, optionally including selective reception of fundamental, harmonic or sub-harmonic echo frequencies; power Doppler imaging, optionally including selective reception of fundamental, harmonic or sub-harmonic echo frequencies; power or colour Doppler imaging utilising loss of correlation or apparent Doppler shifts caused by changes in the acoustical properties of contrast agent microbubbles such as may be caused by spontaneous or ultrasound-induced destruction, fragmentation, growth or coalescence; pulse inversion imaging, optionally including selective reception of fundamental, harmonic or sub-harmonic echo frequencies, and also including techniques where the number of pulses emitted in each direction exceeds two; pulse inversion imaging utilising loss of correlation caused by changes in the acoustical properties of contrast agent microbubbles such as may be caused by spontaneous or ultrasound-induced destruction, fragmentation, growth or coalescence; pulse predistortion imaging, e.g. as described in 1997 IEEE Ultrasonics Symposium, pp. 1567-1570; and ultrasound imaging techniques based on comparison of echoes obtained with different emission output amplitudes or waveform shapes in order to detect non-linear effects caused by the presence of gas microbubbles.

For a given dose of the gas dispersion and diffusible component compositions, the use of colour Doppler imaging ultrasound to induce growth of the dispersed gas has been found to give stronger contrast effects during subsequent B-mode imaging, possibly as a result of the higher ultrasound intensities employed. To reduce the effects of movement, successive images of tissues such as the heart or kidney may be collected with the aid of suitable synchronisation techniques (e.g. gating to the ECG or respiratory movement of the subject). Measurement of changes in resonance frequency or frequency absorption which accompany growth of the dispersed gas may also usefully be made to detect the contrast agent.

It will be appreciated that the dispersed gas content of combined contrast agent preparations according to the invention will tend to be temporarily retained in tissue in concentrations proportional to the regional rate of tissue perfusion. Accordingly, when using ultrasound imaging modalities such as conventional or harmonic B-mode imaging where the display is derived directly from return signal intensities, images of such tissue may be interpreted as perfusion maps in which the displayed signal intensity is a function of local perfusion. This is in contrast to images obtained using free-flowing contrast agents, where the regional concentration of contrast agent and corresponding return signal intensity depend on the actual blood content rather than the rate of perfusion of local tissue.

In cardiac studies, where perfusion maps are derived from return signal intensities in accordance with this embodiment of the invention, it may be advantageous to subject a patient to physical or pharmacological stress in order to enhance the distinction, and thus the difference in image intensities, between normally perfused myocardium and any myocardial regions supplied by stenotic arteries. As is known from radionucleide cardiac imaging, such stress induces vasodilatation and increased blood flow in healthy myocardial tissue, whereas blood flow in underperfused tissue supplied by a stenotic artery is substantially unchanged since the capacity for arteriolar vasodilatation is already exhausted by inherent autoregulation seeking to increase the restricted blood flow.

The application of stress as physical exercise or pharmacologically by administration of adrenergic agonists may cause discomfort such as chest pains in patient groups potentially suffering from heart disease, and it is therefore preferable to enhance the perfusion of healthy tissue by administration of a vasodilator drug. Representative vasodilator drugs useful in accordance with the invention include endogenous/ metabolic vasodilators such as lactic acid, adenosine triphosphate, adenosine diphosphate, adenosine monophosphate, adenosine, nitric oxide and agents causing hypercapnia, hypoxia/hypoxemia or hyperemia; phosphodiesterase inhibitors such as dipyridamole and sildenafil; sympathetic activity inhibitors such as clonidine and methyldopa; smooth muscle relaxants such as papaverine, hydralazine, dihydralazine and nitroprusside; beta recepator agonists such as dopamine, dobutamine, arbutamine, albuterol, salmeterol and isoproterenol; alpha receptor antagonists such as doxazosin, terazosin and prazosin; organic nitrates such as glyceryl trinitrate, isosorbide dinitrate and isosorbide mononitrate; angiotensin converting enzyme (ACE) inhibitors such as benazepril, captopril, enalapril, fosinopril, lisinopril, quinapril and ramipril; angiotensin II antagonists (or AT1 receptor antagonists) such as valsartane, losartan and candesartan; calcium channel blockers such as amlodipine, nicardipine, nimodipine, felodipine, isradipine, diltiazem, verapamil and nifedipine; prostaglandins such as alprostadil; and endothelium-dependent vasodilators.

Use of adenosine is particularly preferred since it is an endogenous substance and has a rapid but short-lived vasodilatating effect. This latter property is confirmed by the fact that it has a blood pool half-life of only a few seconds; possible discomfort to patients during vasodilatation is therefore minimised. Vasodilatation induced by adenosine will be most intense in the heart since the drug will tend to reach more distal tissues in less than pharmacologically active concentrations; it is therefore the vasodilator drug of choice in cardiographic applications of the method of the invention.

In addition to arterial stenoses, other tissue/perfusion abnormalities which affect local vasoregulation may be detectable in accordance with the invention by induction of vasomodification. Thus, for example, vessels with malignant lesions are known to be poorly differentiated and may therefore exhibit impaired response to vasoconstrictor drugs compared to normal tissue; a similar lack of vasoconstrictory response may occur in severely inflamed tissue. Observation of the response to a vasoconstrictor stimulus in terms of changes in signal intensity during an imaging procedure may therefore give useful diagnostic information. Representative examples of vasoconstrictor drugs which may be useful in such embodiments include isoprenaline, epinephrine, norepinephrine, dopamine, metaraminol, prenalterol, ergotamine, dihydroergotamine, methysergide and inhibitors of nitric acid production such as analogues of L-arginine; such drugs may, for example, be administered either locally or systemically.

For some purposes it may be advantageous to administer two or more vasoactive substances, either together or in sequence. Where two vasoactive substances are applied, both may be vasodilators, both may be vasoconstrictors, or one may be a vasodilator and the other a vasoconstrictor. When two vasodilators or two vasoconstrictors are used, they should differ in at least one property, e.g. tissue specificity or mechanism of action, so that local differences in signal intensity may be determined during a single examination. When administered separately, a vasoconstrictor may be administered first, followed by a vasodilator, or the reverse order may be used.

Administration of adenosine may lead to in excess of fourfold increases in coronary blood flow in healthy myocardial tissue, greatly increasing the uptake and temporary retention of contrast agents in accordance with the invention and thus significantly increasing the difference in return signal intensities between normal and hypoperfused myocardial tissue. Because an essentially physical entrapment process is involved, retention of contrast agents according to the invention is highly efficient; this may be compared to the uptake of radionucleide tracers such as thallium 201 and technetium sestamibi, which is limited by low contact time between tracer and tissue and so may require maintenance of vasodilatation for the whole period of blood pool distribution for the tracer (e.g. 4-6 minutes for thallium scintigraphy) to ensure optimum effect. The contrast agents of the invention, on the other hand, do not suffer such diffusion or transport limitations, and since their retention in myocardial tissue may also rapidly be terminated, for example by cessation of growth-generating ultrasound irradiation, the period of vasodilatation needed to achieve cardiac perfusion imaging in accordance with this embodiment of the invention may be very short, for example less than one minute. This will reduce the duration of any possible discomfort caused to patients by administration of vasodilator drugs.

It will be appreciated that because of the short half-life of adenosine noted above, its repeated injection or infusion may be necessary during cardiac imaging in accordance with this embodiment of the invention; by way of example, an initial administration of 150 µg/kg of adenosine may be made substantially simultaneously with administration of the contrast agent composition, followed 10 seconds later by slow injection of a further 150 µg/kg of adenosine, e.g. over a period of 20 seconds. An infusion of adenosine at a constant rate during the time interval covering injection and deposition of contrast agent in the myocardium may also be used.

Contrast agent preparations in accordance with the invention may advantageously be employed as delivery agents for bioactive moieties such as therapeutic drugs (i.e. agents having a beneficial effect on a specific disease in a living human or non-human animal), particularly to targeted sites. Thus, for example, therapeutic compounds may be present in the first composition, e.g. in the dispersed gas, linked to part of the stabilising material (e.g. through covalent or ionic bonds, if desired through a spacer arm), or physically mixed into such stabilising material; this last option is particularly applicable where the therapeutic compound and stabilising material have similar polarities or solubilities.

The controllable growth properties of the dispersed gas may be utilised to bring about its temporary retention in the microvasculature of a target region of interest; use of ultrasonic irradiation to induce growth and thus retention of the gas and associated therapeutic compound in a target structure is particularly advantageous. Localised injection of the gas-containing first composition or, more preferably, the diffusible component-containing second composition, e.g. as hereinbefore described, may also be used to concentrate growth of the dispersed gas in a target area.

The therapeutic compound, which may if desired be coupled to a site-specific vector having affinity for specific cells, structures or pathological sites, may be released as a result of, for example, stretching or fracture of the gas-stabilising material caused by growth of the dispersed gas, solubilisation of the stabilising material, or disintegration of gas-microbubbles or gas-containing microparticles (e.g. induced by ultra-sonication or by a reversal of the concentration gradient of the diffusible component in the target area). Where a therapeutic agent is chemically linked to the gas-stabilising material, the linkage or any spacer arm associated therewith may advantageously contain one or more labile groups which are cleavable to release the agent. Representative cleavable groups include amide, imide, imine, ester, anhydride, acetal, carbamate, carbonate, carbonate ester and disulphide groups which are biodegradable in vivo, e.g. as a result or hydrolytic and/or enzymatic action.

Representative and non-limiting examples of drugs useful in accordance with this embodiment of the invention include antineoplastic agents such as vincristine, vinblastine, vindesine, busulfan, chlorambucil, spiroplatin, cisplatin, carboplatin, methotrexate, adriamycin, mitomycin, bleomycin, cytosine arabinoside, arabinosyl adenine, mercaptopurine, mitotane, procarbazine, dactinomycin (antinomycin D), daunorubicin, doxorubicin hydrochloride, taxol, plicamycin, aminoglutethimide, estramustine, flutamide, leuprolide, megestrol acetate, tamoxifen, testolactone, trilostane, amsacrine (m-AMSA), asparaginase (L-asparaginase), etoposide, interferon a-2a and 2b, blood products such as hematoporphyrins or derivatives of the foregoing; biological response modifiers such as muramylpeptides; antifungal agents such as ketoconazole, nystatin, griseofulvin, flucytosine, miconazole or amphotericin B; hormones or hormone analogues such as growth hormone, melanocyte stimulating hormone, estradiol, beclomethasone dipropionate, betamethasone, cortisone acetate, dexamethasone, flunisolide, hydrocortisone, methylprednisolone, paramethasone acetate, prednisolone, prednisone, triamcinolone or fludrocortisone acetate; vitamins such as cyanocobalamin or retinoids; enzymes such as alkaline phosphatase or manganese superoxide dismutase; antiallergic agents such as amelexanox; anticoagulation agents such as warfarin, phenprocoumon or heparin; antithrombotic agents; circulatory drugs such as propranolol; metabolic potentiators such as glutathione; antituberculars such as p-aminosalicylic acid, isoniazid, capreomycin sulfate, cyclosexine, ethambutol, ethionamide, pyrazinamide, rifampin or streptomycin sulphate; antivirals such as acyclovir, amantadine, azidothymidine, ribavirin or vidarabine; blood vessel dilating agents such as diltiazem, nifedipine, verapamil, erythritol tetranitrate, isosorbide dinitrate, nitroglycerin or pentaerythritol tetranitrate; antibiotics such as dapsone, chloramphenicol, neomycin, cefaclor, cefadroxil, cephalexin, cephradine, erythromycin, clindamycin, lincomycin, amoxicillin, ampicillin, bacampicillin, carbenicillin, dicloxacillin, cyclacillin, picloxacillin, hetacillin, methicillin, nafcillin, penicillin or tetracycline; antiinflammatories such as diflunisal, ibuprofen, indomethacin, meclefenamate, mefenamic acid, naproxen, phenylbutazone, piroxicam, tolmetin, aspirin or salicylates; antiprotozoans such as chloroquine, metronidazole, quinine or meglumine antimonate; antirheumatics such as penicillamine; narcotics such as paregoric; opiates such as codeine, morphine or opium; cardiac glycosides such as deslaneside, digitoxin, digoxin, digitalin or digitalis; neuromuscular ,blockers such as atracurium mesylate, gallamine triethiodide, hexafluorenium bromide, metocurine iodide, pancuronium bromide, succinylcholine chloride, tubocurarine chloride or vecuronium bromide; sedatives such as amobarbital, amobarbital sodium, apropbarbital, butabarbital sodium, chloral hydrate, ethchlorvynol, ethinamate, flurazepam hydrochloride, glutethimide, methotrimeprazine hydrochloride, methyprylon, midazolam hydrochloride, paraldehyde, pentobarbital, secobarbital sodium, talbutal, temazepam or triazolam; local anaesthetics such as bupivacaine, chloroprocaine, etidocaine, lidocaine, mepivacaine, procaine or tetracaine; general anaesthetics such as droperidol, etomidate, fentanyl citrate with droperidol, ketamine hydrochloride, methohexital sodium or thiopental and pharmaceutically acceptable salts (e.g. acid addition salts such as the hydrochloride or hydrobromide or base salts such as sodium, calcium or magnesium salts) or derivatives (e.g. acetates) thereof; and radiochemicals, e.g. comprising beta-emitters. Of particular importance are antithrombotic agents such as heparin and agents with heparin-like activity such as antithrombin III, dalteparin and enoxaparin; blood platelet aggregation inhibitors such as ticlopidine, aspirin, dipyridamole, iloprost and abciximab; and thrombolytic enzymes such as streptokinase and plasminogen activator. Other examples of therapeutics include genetic material such as nucleic acids, RNA, and DNA of natural or synthetic origin, including recombinant RNA and DNA. DNA encoding certain proteins may be used in the treatment of many different types of diseases. For example, tumour necrosis factor or interleukin-2 may be provided to treat advanced cancers; thymidine kinase may be provided to treat ovarian cancer or brain tumors; interleukin-2 may be provided to treat neuroblastoma, malignant melanoma or kidney cancer; and interleukin-4 may be provided to treat cancer.

Contrast agent preparations in accordance with the invention may be used as vehicles for contrast-enhancing moieties for imaging modalities other than ultrasound, for example X-ray, light imaging, magnetic resonance and, more preferably, scintigraphic imaging agents. Controlled growth of the dispersed gas phase may be used to position such agents in areas of interest within the bodies of subjects, for example using ultrasound irradiation of a target organ or tissue to induce the desired controlled growth and temporary retention of the agent, which may then be imaged using the appropriate non-ultrasound imaging modality.

Contrast agent preparations in accordance with the invention may also be used as vehicles for therapeutically active substances which do not necessarily require release from the preparation in order to exhibit their therapeutic effect. Such preparations may, for example, incorporate radioactive atoms or ions such as beta-emitters which exhibit a localised radiation-emitting effect following growth of the dispersed gas phase and temporary retention of the agent at a terget site. It will be appreciated that such agents should preferably be designed so that subsequent shrinkage and cessation of retention of the dispersed gas does not occur until the desired therapeutic radiation dosage has been administered.

Contrast agent preparations in accordance with the invention may additionally exhibit therapeutic properties in their own right. Thus, for example, preparations may be used therapeutically by intravenously injecting a high dose of the agent and then exposing an artery leading to a tumour to local ultrasound irradiation. The growing gas phase may then block blood circulation to the tumour. Thus it is possible by applying localised ultrasonic energy to obtain a controlled and localised embolism; this may be of importance as such or in combination with other therapeutic measures. Concentrations of dispersed gas in capillaries may also enhance absorption of ultrasonic energy in hyperthermic therapy; this may be used in,for example, treatment of liver tumours. Other tissues which may be treated in this way include breast, thyroid and prostate. Irradiation with a relatively high energy (e.g. 5 W) focused ultrasound beam, e.g. at 1.5 MHz, may be appropriate in such applications.

The following non-limitative Examples serve to illustrate the invention.

### Preparation 1

### Perfluorobutane gas dispersion with negatively charged surface material

Hydrogenated phosphatidylserine (5 mg/ml in a 1% w/w solution of propylene glycol in purified water) and perfluorobutane gas were homogenised in-line at 7800 rpm and ca. 40°C to yield a creamy-white microbubble dispersion. The dispersion was fractionated to substantially remove undersized microbubbles (<2 µm) and the volume of the dispersion was adjusted to the desired microbubble concentration by adding aqueous sucrose to give a sucrose concentration of 92 mg/ml. 2 ml portions of the resulting dispersion were filled into 10 ml flat-bottomed vials specially designed for lyophilisation, and the contents were lyophilised to give a white porous cake. The lyophilisation chamber was then filled with perfluorobutane and the vials were sealed. Prior to use, water was added to a vial and the contents were gently hand-shaken for several seconds to give a perfluorobutane microbubble dispersion; the concentration of microbubbles in the dispersion was 1.1% v/v and the median microbubble size was 2.7 µm.

### Preparation 2

### Perfluorobutane gas dispersion with positively charged surface material

1 ml of a dispersion of 1,2-distearoyl-3-trimethylammoniumpropane (1 mg/ml) and distearoylphosphatidylcholine (4 mg/ml) in a 2% w/v solution of propylene glycol in purified water was placed in a 2 ml vial. The headspace was flushed with perfluorobutane gas and the vial was then closed and shaken for 45 seconds using an Espe CapMix® mixer for dental materials. The resulting milky white microbubble dispersion was washed three times by centrifugation and removal of infranatant, whereafter an equal volume of purified water was added. The concentration of microbubbles in the resulting dispersion was 4.9% v/v and the median microbubble size was 3.2 µm.

### Preparation 3

### Perfluorobutane gas dispersion with biotinylated surface material

Distearoylphosphatidylserine (4.5 mg) and biotin-dipalmitoylphosphatidylethanolamine (0.5 mg) were weighed into a clean vial and 1.0 ml of a solution of 1.4% propylene glycol/2.4% glycerol was added. Following heating to 78°C the mixture was cooled to room temperature and the head space was flushed with perfluorobutane gas. The vial was closed, shaken for 45 seconds using an Espe CapMix® mixer, and then placed on a roller table for 16 hours. The resulting microbubble dispersion was washed extensively with deionised water.

### Preparation 4

### Perfluorodimethylcyclobutane emulsion with positively charged surface material

1 ml of a dispersion of didodecyldimethylammonium bromide (5 mg/ml in purified water) was placed in a 2 ml vial to which was added 100 µl of perfluorodimethylcyclobutane (b.p. 45°C). The vial was closed and then shaken for 75 seconds using an Espe CapMix® to yield an emulsion of diffusible component which was stored at 0°C when not in use. The emulsion was washed three times by centrifugation and removal of the infranatant followed by addition of an equivalent volume of purified water. The concentration of droplets in the emulsion was 6.2% v/v and the median droplet size was 2.3 µm.

### Preparation 5

### Perfluorohexane emulsion with positively charged surface material

1 ml of a dispersion of 1,2-distearoyl-3-trimethylammoniumpropane (1 mg/ml) and distearoylphosphatidylcholine (4 mg/ml) in purified water was placed in a 2 ml vial to which was added 100 µl of perfluorohexane (b.p. 57°C). The vial was closed and then shaken for 75 seconds using an Espe CapMix® to yield an emulsion of diffusible component which was stored at 0°C when not in use. The emulsion was washed three times by centrifugation and removal of the infranatant followed by addition of an equivalent volume of purified water. The concentration of droplets in the emulsion was 2.9% v/v and the median droplet size was 2.9 µm.

### Preparation 6

### Perfluorodimethylcyclobutane emulsion with negatively charged surface material

1 ml of a dispersion of hydrogenated phosphatidylserine (5 mg/ml in purified water) was placed in a 2 ml vial to which was added 100 µl of perfluorodimethylcyclobutane (b.p. 45°C). The vial was closed and then shaken for 75 seconds using an Espe CapMix® to yield an emulsion of diffusible component which was stored at 0°C when not in use. The emulsion was washed three times by centrifugation and removal of the infranatant followed by addition of an equivalent volume of purified water. The concentration of droplets in the emulsion was 6.9% v/v and the median droplet size was 2.7 µm.

### Preparation 7

### Perfluorodimethylcyclobutane emulsion with avidinylated surface material

Distearoylphosphatidylserine (4.5 mg) and biotin-dipalmitoylphosphatidylethanolamine (0.5 mg) were weighed into a clean vial and 1.0 ml of a solution of 2% propylene glycol was added. Following heating to 80°C the mixture was cooled to room temperature. 100 µl of perfluorodimethylcyclobutane were added and the vial was closed and shaken for 75 seconds using an Espe CapMix® to yield an emulsion of diffusible component. A diluted sample of the emulsion (100 µl emulsion in 1 ml water) was incubated with excess avidin and placed on a roller table. The diluted emulsion was then washed extensively with water and concentrated by centrifuging.

### Preparation 8

### Perfluorodimethylcyclobutane emulsion with positively charged surface material

1,2-Distearoyl-3-trimethylammoniumpropane (73 mg) and distearoylphosphatidylcholine (641 mg) were placed in a 250 ml round bottom flask and chloroform (100 ml) was added. The flask was heated under hot tap water until a clear solution was obtained, whereafter the flask was put on a rotavapor and the chloroform was removed by evaporation at 350 mbar using a bath temperature of 45°C. In order to remove residual traces of solvent the sample was exposed to ca. 20 mbar vacuum overnight. Thereafter, MilliQ water (143 ml) was added and the flask was again placed on a rotavapor and rotated at full speed while immersed into a 80°C water bath. After ca. 25 minutes the sample was transferred to a suitable vial and placed in a refrigerator for cooling overnight.

1 ml portions of the sample were transferred to 2 ml chromatography vials and 100 µl of perfluorodimethylcyclobutane (b.p. 45°C) was added to each vial. The vials were shaken on an Espe CapMix® for 75 seconds and the samples were immediately cooled on ice. The contents of the vials were collected in a larger vial and the emulsion was characterised with respect to size distribution and total particle volume concentration using a Coulter counter; the median droplet size was 2.67 µm, confirming that the emulsion was acceptable for injection. The particle volume concentration measurement was used to adjust the concentration to ca. 1% v/v disperse phase using MilliQ water. The emulsion was stored in a refrigerator until use.

### Preparation 9

### Perfluoromethylcyclopentane emulsion with positively charged surface material

The procedure of Preparation 8 was repeated except that perfluoromethylcyclopentane (b.p. 48°C) was used in place of perfluorodimethylcyclobutane. Coulter counter analysis showed the median droplet size of the emulsion to be 2.63 µm.

### Preparation 10

### Perfluoro-2-methylpentane emulsion with positively charged surface material

The procedure of Preparation 8 was repeated except that perfluoro-2-methylpentane (b.p. 50-57°C) was used in place of perfluorodimethylcyclobutane. Coulter counter analysis showed the median droplet size of the emulsion to be 2.72 µm.

### Preparation 11

### Perfluorohexane emulsion with positively charged surface material

The procedure of Preparation 8 was repeated except that perfluorohexane (b.p. 58-60°C) was used in place of perfluorodimethylcyclobutane. Coulter counter analysis showed the median droplet size of the emulsion to be 2.54 µm.

### Preparation 12

### Synthesis of the positively charged lipopeptide: palmitoyl-Lys(palmitoyl)-Lys-Lys-Ahx-Lys-Arg-Lys-Arg-Lys-Arg-NH₂ (where Ahx = aminohexanoic acid)

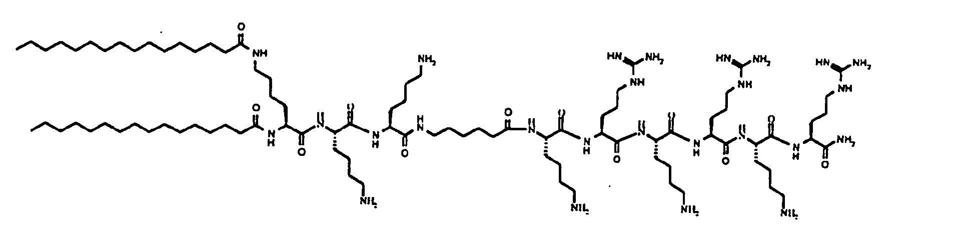

The lipopeptide was synthesised on an ABI 433A automatic peptide synthesiser starting with Rink amide resin on a 0.25 mmol scale, using 1 mmol amino acid cartridges. All amino acids and palmitic acid were preactivated using O-benzotriazol-1-yl-N,N,N',N'-tetramethyluronium hexafluorophosphate (HBTU) before coupling. The simultaneous removal of peptide and side-chain protecting groups from the resin was carried out in trifluoroacetic acid (TFA) containing 5% phenol, 5% triisopropylsilane and 5% water for 2 hours, giving a crude product yield of 150 mg. Purification by preparative HPLC (Vydac 218TP1022 column) of a 30 mg aliquot of crude material was carried out using a gradient of 70 to 100% B over 40 minutes (A = 0.1% TFA/water and B = acetonitrile) at a flow rate of 9 ml/min. After lyophilization, 19 mg of pure material was obtained (analytical HPLC: gradient 70-100% B where B = acetonitrile and A = 0.01% TFA/water; column - Vydac 218TP54: detection - UV 214 nm; product retention time = 11 minutes). Further product characterisation was carried out using MALDI mass spectrometry; expected M+H at 1845, found at 1850.

### Preparation 13

### Synthesis of the positively charged lipopeptide: palmitoyl-Dpr(palmitoyl)-Arg-Arg-Lys-NH₂ (where Dpr = diaminopropionic acid)

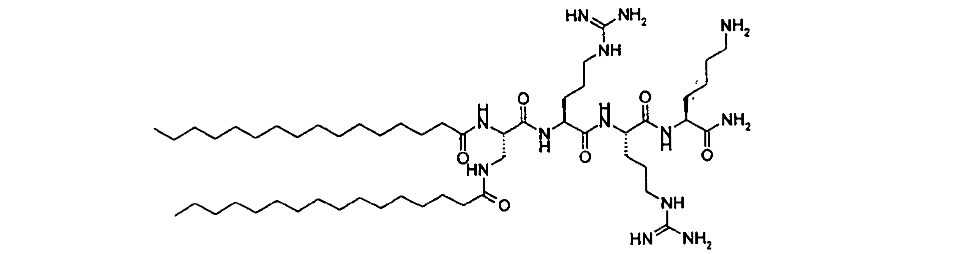

The lipopeptide was synthesised on an ABI 433A automatic peptide synthesiser starting with Rink amide resin on a 0.25 mmol scale, using 1 mmol amino acid cartridges. All amino acids and palmitic acid were preactivated using HBTU before coupling. The simultaneous removal of peptide and side-chain protecting groups from the resin was carried out in TFA containing 5% phenol, 5% triisopropylsilane and 5% water for 2 hours, giving a crude product yield of 50 mg. Purification by preparative HPLC (Vydac 218TP1022 column) of crude material was carried out using a gradient of 90 to 100% B over 40 minutes (A = 0.1% TFA/water and B = 0.1% TFA/acetonitrile) at a flow rate of 9 ml/min. After lyophilization, 5 mg of pure material were obtained (analytical HPLC: gradient 80-100% B where A = 0.1% TFA/water and B = 0.1% TFA/acetonitrile; column - Vydac 218TP54; detection - UV 214 nm; product retention time = 15 minutes). Further product characterisation was carried out using MALDI mass spectrometry: expected M+H at 1021, found at 1022.

### Preparation 14

### a) Hexadecanoic acid 2-tert-butoxycarbonylaminoethyl ester

N-Boc-ethanolamine (1.6g, 10 mmol) and palmitoyl chloride (3.28g, 12 mmol) were dissolved in dichloromethane (25 ml) and triethylamine (1.68 ml, 12 mmol) was added with stirring. The reaction mixture was stirred at room temperature overnight. The reaction mixture was diluted to 100 ml with dichloromethane, transferred to an extraction vessel, washed with 1 x 10 ml 1M sodium hydrogen carbonate and 2 x 25 ml water and dried, whereafter the solvent was removed *in vacuo.* The crude product was purified by column chromatography on silica. Identity: TLC (one spot) and MALDI (M+1).

### b) Hexadecanoic acid 2-aminoethyl ester hydrochloride

Hexadecanoic acid 2-tert-butoxycarbonylaminoethyl ester (1.1g, 2.7mmol) from (a) above was dissolved in 4M hydrogen chloride/dioxane (10 ml) with stirring. A white precipitate started to form after a few minutes. TLC showed full conversion of starting material after 30 minutes. The white precipitate was collected by filtration, washed on the filter with dioxane and dried *in vacuo.* Identity: TLC (one spot) and MALDI (M+1).

### Preparation 15

### a) 4-Hexadecanoylaminobutylcarbamic acid tert-butyl ester

Boc-1,4-diaminobutane (1g, 5.3mmol) and palmitoyl chloride (1.64g, 6mmol) were dissolved in dichloromethane (25 ml). Triethylamine (0.64ml, 6 mmol) was added and the reaction mixture was stirred overnight, then diluted to 150 ml with dichloromethane, transferred to an extraction funnel, washed with:1 x 10 ml 1M sodium hydrogen carbonate and 2 x 25 ml water and dried, whereafter the solvent was removed *in vacuo.* The crude product was dissolved in chloroform (25 ml) and placed in a refrigerator overnight. The pure product was isolated as sticky crystals. Identity: TLC (one spot) and MALDI (M+1).

### b) Hexadecanoic acid 4-aminobutyl amide hydrochloride

4-Hexadecanoylaminobutylcarbamic acid tert-butyl ester (1g, 2.3mmol) from (a) above was dissolved in 4M hydrogen chloride/dioxane (10 ml) with stirring. Precipitation of white crystals started after a few minutes. The reaction mixture was diluted with dioxane (10 ml) and stirring was continued for 4 hours, at which time TLC showed full conversion of starting material. The white precipitate was collected by filtration, washed on the filter with dioxane and dried *in vacuo.* Identity: TLC (one spot) and MALDI (M+1).

### Preparation 16

### a) tert-Butoxycarbonylaminoacetic acid hexadecyl ester

Boc-Gly-OH (1.74g, 10 mmol) and 1-hexadecanol (2.5g, 10mmol) were dissolved in dichloromethane (30 ml) and dimethylaminopyridine (30 mg, catalytic amount) was added. Dicyclohexylcarbodiimide (2.1g, 10 mmol) dissolved in dichloromethane (10 ml) was added dropwise over 10 minutes with stirring and the reaction mixture was stirred at room temperature overnight. Precipitated dicyclohexylurea was removed by filtration and the organic phase was diluted to 150 ml with dichloromethane. The organic phase was extracted with 1 × 5 ml 1M sodium hydrogen carbonate and 2 × 10 ml water and dried, whereafter the solvent was removed in *vacuo.* The crude product was used in the next step without further purification. Identity: TLC (one spot) and MALDI (M+1).

### b) Aminoacetic acid hexadecyl ester hydrochloride

tert-Butoxycarbonylaminoacetic acid hexadecyl ester (2g, 5mmol) from (a) above was dissolved in dioxane (20 ml). 4M hydrogen chloride/dioxane (10 ml) was added and the reaction mixture was stirred at room temperature. After 30 minutes, a white precipitate started to form. Diethylether (50 ml) was added and the reaction mixture was stirred at room temperature overnight, whereafter the precipitate was collected by filtration and washed with diethyl ether. TLC showed full conversion of the starting material, but the product was contaminated by a small amount of 1-hexadecanol. The pure product was produced by column chromatography on silica. Identity: TLC (one spot) and MALDI (M+1).

### Preparation 17

### Methylaminoacetic acid hexadecyl ester hydrochloride

4M hydrogen chloride/dioxane (10 ml) was added to a reaction vessel containing N-methylglycine (100mg, 1.1mmol) and 1-hexadecanol (1g, 4.1mmol). The slurry was stirred at room temperature for several days. After 4 days the reaction mixture was homogenous and TLC showed full conversion of the amino acid. The solvent was removed *in vacuo* and the crude product was purified by column chromatography on silica. Identity: TLC (one spot) and MALDI (M+1).

### Preparation 18

### Dimethylaminoacetic acid hexadecyl ester hydrochloride

4M hydrogen chloride/dioxane (10 ml) was added to a reaction vessel containing N,N-dimethylglycine hydrochloride (150 mg, 1.1mmol) and 1-hexadecanol (1.33g, 5.5mmol). The slurry was stirred at room temperature. After 3 weeks the reaction mixture was homogenous and TLC showed full conversion of the amino acid. The solvent was removed *in vacuo* and the crude product was purified by column chromatography on silica. Identity: TLC (one spot) and MALDI (M+1).

### Preparations 19 - 36

### Emulsions with positively charged surface material

Distearoylphosphatidylcholine (90 mg) and a cationic additive from Table 1 below (10 mg) were placed in a 50 ml round bottom flask and chloroform (10 ml) was added. [In Preparation 31, methanol (1 ml) was added to the chloroform in order to dissolve the components]. The flask was heated under hot tap water until a clear solution was obtained, whereafter the flask was put on a rotavapor and the chloroform was removed by evaporation at 350 mbar using a bath temperature of 45°C. In order to remove residual traces of solvent the sample was exposed to *ca.* 20 mbar vacuum overnight. Thereafter, MilliQ water (20 ml) was added and the flask was again placed on a rotavapor and rotated at full speed while immersed into a 80°C water bath. After ca. 10 minutes the sample was transferred to a suitable vial and placed in a refrigerator for cooling overnight.

1 ml portions of each sample were transferred to 2 ml chromatography vials and perfluorodimethylcyclobutane (100 µl) was added to each vial. The vials were shaken on an Espe CapMix® for 75 seconds, and the samples were immediately cooled on ice. The emulsions were collected in larger vials and were characterised with respect to size distribution and total particle volume concentration using a Coulter counter; the median droplet sizes are given in the following Table 1. The particle volume concentration measurements were used to adjust the concentration of each emulsion to ca. 1% v/v disperse phase using MilliQ water. The emulsions were stored in a refrigerator until use.

**Table 1**

| **Prepn. No.** | **Cationic additive** | **Median droplet size (µm)** |
|---|---|---|
| 19 | DC-Cholesterol | 3 |
| 20 | 1,2-Distearoyl ethylphosphocholine | 2.4 |
| 21 | Benzylcetyldimethylammonium chloride | 2.4 |
| 22 | Cetyltrimethylammonium bromide | 2.6 |
| 23 | Cetylpyridinium chloride | 2.5 |
| 24 | Palmitoyl-Dpr(palmitoyl)-Arg-Arg-Lys-NH₂ (Prepn. 13) | 3.6 |
| 25 | Myristoyl choline chloride | 2.9 |
| 26 | Hexadecanoic acid 2-aminoethyl ester (Prepn. 14) | 2.5 |
| 27 | Hexadecanoic acid 4-aminobutyl amide (Prepn. 15) | 2.3 |
| 28 | Aminoacetic acid hexadecyl ester (Prepn. 16) | 2.4 |
| 29 | Cetyl carnitine ester | 2.4 |
| 30 | Psycosine | 2.5 |
| 31 | D-Sphingosine sulphate | 2.7 |
| 32 | Phytosphingosine | 2.4 |
| 33 | DL-Dihydrosphingosine | 2.9 |
| 34 | Didodecyldimethylammonium bromide | 2.4 |
| 35 | Methylaminoacetic acid hexadecyl ester (Prepn. 17) | 3.1 |
| 36 | Dimethylaminoacetic acid hexadecyl ester (Prepn. 18) | 3.5 |

### Preparation 37

### Perfluorodimethylcyclobutane emulsion containing the positively charged lipopeptide palmitoyl-Lys (palmitoyl)-Lys-Lys-Ahx-Lys-Arg-Lys-Arg-Lys-Arg-NH₂ (where Ahx = aminohexanoic acid)

Distearoylphosphatidylcholine (90 mg) and the positively charged lipopeptide palmitoyl-Lys(palmitoyl)-Lys-Lys-Ahx-Lys-Arg-Lys-Arg-Lys-Arg-NH₂ from Preparation 12 (10 mg) are placed in a 50 ml round bottom flask and chloroform (10 ml)) is added. The flask is heated under hot tap water until a clear solution is obtained, whereafter the flask is put on a rotavapor and the chloroform is removed by evaporation. In order to remove residual traces of solvent the sample may be exposed to vacuum overnight. Thereafter, MilliQ water (20 ml) is added and the flask is again placed on a rotavapor and rotated at full speed while immersed into a 80°C water bath. After ca. 10 minutes the sample is transferred to a suitable vial and placed in a refrigerator for cooling overnight.

1 ml portions of the sample are transferred to 2 ml chromatography vials and 100 µl of perfluorodimethylcyclobutane is added to each vial. The vials are shaken on an Espe CapMix® for 75 seconds and the samples are immediately cooled on ice. The contents of each vial are collected in a larger vial and the emulsion is characterised with respect to size distribution and total particle volume concentration using a Coulter counter. The particle volume concentration measurement is used to adjust the concentration to ca. 1% v/v disperse phase using MilliQ water. The emulsion is stored in a refrigerator until use.

### Example 1

### In vivo imaging of dog heart

A 20 kg mongrel dog was anaesthetised, a mid-line sternotomy was performed, and the anterior pericardium was removed. Mid-line short-axis B-mode imaging of the heart was performed through a low-attenuating 30 mm silicone rubber spacer, using an ATL HDI-3000 scanner equipped with a P3-2 transducer. The framerate was 40 Hz and the mechanical index was 1.1.

### a) [Comparative] Imaging using negatively charged perfluorobutane gas dispersion nd negatively charged perfluorodimethylcyobutane emulsion

An amount of the perfluorobutane gas dispersion from Preparation 1 corresponding to 0.2 µl gas/kg body weight and an amount of the perfluorodimethylcyclobutane emulsion from Preparation 6 corresponding to 0.4 µl perfluorodimethylcyclobutane/kg body weight were simultaneously injected intravenously into the dog. A substantial rise in echo intensity from the myocardium was seen, starting 20 seconds after the injection and lasting for 10 minutes. Clearance of contrast agent effects from the blood pool occurred earlier than loss of myocardial contrast effect.

### b) Imaging using negatively charged perfluorobutane gas dispersion and positively charged perfluorodimethylcyclobutane emulsion (high dose)

An amount of the perfluorobutane gas dispersion from Preparation 1 corresponding to 0.2 µl gas/kg body weight and an amount of the perfluorodimethylcyclobutane emulsion from Preparation 4 corresponding to 0.1 µl perfluorodimethylcyclobutane/kg body weight were simultaneously injected intravenously into the dog. The resulting myocardial contrast effect was far more intense than that observed in (a) above and lasted for 20 minutes.

### c) Imaging using negatively charged perfluorobutane gas dispersion and positively charged perfluorodimethylcyclobutane emulsion (low dose)

The procedure described in Example 1(b) was repeated except that the dose of the perfluorodimethylcyclobutane emulsion was reduced to an amount corresponding to 0.02 µl perfluorodimethylcyclobutane/kg body weight. The resulting myocardial contrast effect was comparable to that observed in Example 1(a).

### Example 2

### In vivo imaging of dog heart (low contrast agent dose)

### a) Imaging using negatively charged perfluorobutane gas dispersion and positively charged perfluorohexane emulsion

The procedure described in Example 1(b) was repeated except that the perfluoromethylcyclobutane emulsion was replaced by an amount of the perfluorohexane emulsion from Preparation 5 corresponding to 0.02 µl perfluorohexane/kg body weight. The resulting myocardial contrast effect was comparable to that observed in Example 1(a).

### b) [Comparative] Imaging using negatively charged perfluorobutane gas dispersion and negatively charged perfluorodimethylcyclobutane emulsion

The procedure described in Example 1(a) was repeated except that the dose of the perfluorodimethylcyclobutane emulsion was reduced to an amount corresponding to 0.02 µl perfluorodimethylcyclobutane/kg body weight. Only faint myocardial contrast effects could be seen.

### c) [Comparative] Imaging using positively charged perfluorobutane gas dispersion and positively charged perfluorohexane emulsion

An amount of the perfluorobutane gas dispersion from Preparation 2 corresponding to 0.2 µl gas/kg body weight and an amount of the perfluorohexane emulsion from Preparation 5 corresponding to 0.02 µl perfluorohexane/kg body weight were simultaneously injected intravenously into the dog, imaging as in Example 1. Only faint myocardial contrast effects could be seen.

### d) Imaging using positively charged perfluorobutane gas dispersion and negatively charged perfluorodimethylcyclobutane emulsion

An amount of the perfluorobutane gas dispersion from Preparation 2 corresponding to 0.2 µl gas/kg body weight and an amount of the perfluorodimethylcyclobutane emulsion from Preparation 6 corresponding to 0.02 µl perfluorohexane/kg body weight were simultaneously injected intravenously into the dog, imaging as in Example 1. The resulting myocardial contrast effect was comparable to that observed in Example 1(a).

### Example 3 [Comparative]

### Imaging of dog heart using positively charged perfluorohexane emulsion only_

An amount of the perfluorohexane emulsion from Preparation 5 corresponding to 0.02 µl perfluorohexane/kg body weight was injected intravenously into the dog, imaging as in Example 1. No blood pool or myocardial contrast effects could be seen.

### Example 4 [Comparative]

### Imaging of dog heart without preliminary ultrasonication

The procedure of Example 1(c) was repeated except that the ultrasound scanner was switched off for the first 2 minutes following injection. The contrast effect in the myocardium after the scanner was switched on again was very brief, comparable to that seen at the same time and with the same imaging modality following injection of perfluorobutane gas dispersion alone.

### Example 5

### Imaging of dog heart using biotinylated perfluorobutane gas dispersion and avidinylated perfluorodimethylcyclobutane emulsion

An amount of the perfluorobutane gas dispersion from Preparation 3 corresponding to 0.02 µl gas/kg body weight and an amount of the perfluorodimethylcyclobutane emulsion from Preparation 7 corresponding to 0.02 µl perfluorodimethylcyclobutane/kg body weight were simultaneously injected intravenously into the dog.

Imaging of the heart was performed with a Vingmed CFM-750 sanner, using a midline short axis projection. The scanner was adjusted to maximise ultrasound exposure to the imaged tissue region by using a combination of continuous high frame rate imaging and the highest output power (7 on a scale ranging from 0 to 7). After the injection, initial contrast enhancement was seen in both ventricles of the heart. A steady rise in contrast enhancement was seen in all regions of the myocardium, up to an enhancement intensity approaching the maximum white level on the screen. The duration of tissue contrast was approximately 30 minutes, whilst contrast effects in the blood-pool declined to near baseline within 5 minutes of the injection, leaving an image with almost no blood-pool attenuation, and a complete and extremely bright circumferential contrast enhancement of the myocardium. The contrast effect in the myocardium close to the transducer did not seem to fade despite continuous high intensity ultrasound exposure.

### Examples 6-9

### Imaging of dog heart using negatively charged perfluorobutane gas dispersion and positively charged emulsions from Preparations 8-11

A 19 kg mongrel dog was anaesthetised, a mid-line sternotomy was performed, and the anterior pericardium was removed. Mid-line short-axis B-mode imaging of the heart was performed through a low-attenuating 30 mm silicone rubber spacer, using an ATL HDI-3000 scanner equipped with a P3-2 transducer. The framerate was 40 Hz and the mechanical index was 1.1. An amount of the perfluorobutane gas dispersion from Preparation 1 corresponding to 0.2 µl gas/kg body weight and an amount of one of the emulsions from Preparations 8-11 above corresponding to 0.02 µl volatile oil/kg body weight were simultaneously injected intravenously into the dog. A substantial rise in echo intensity from the myocardium was seen in each case, starting 20 seconds after the injection; the peak intensity was above that observed in Example 1(a).

The ultrasound intensity in the myocardium 90 seconds after injection was corrected for baseline and the resulting myocardial contrast enhancements (MCEs) are given in the following Table 2.

**Table 2**

| **Ex. No.** | **Diffusible component** | **Baseline-corrected MCE (dB)** |
|---|---|---|
| 6 | Perfluorodimethylcyclobutane | 7.9 |
| 7 | Perfluoromethylcyclopentane | 4.8 |
| 8 | Perfluoro-2-methylpentane | 6.6 |
| 9 | Perfluorohexane | 7.5 |

A substantial increase in myocardial opacification was seen at a time when the ventricles were almost emptied of contrast, indicating that the observed contrast enhancement is due to microbubbles retarded in the myocardium. The contrast effect duration varied from ca. 5 to ca. 20 minutes, and was dependent on factors such as the water solubility and vapour pressure of the volatile oil. The following Table 3 shows the MCE half-times for each experiment.

**Table 3**

| **Ex. No.** | **Diffusible component** | **MCE halftime** |
|---|---|---|
| | | **(minutes)** |
| 6 | Perfluorodimethylcyclobutane | 2.9* |
| 7 | Perfluoromethylcyclopentane | 1.9 |
| 8 | Perfluoro-2-methylpentane | 6.9 |
| 9 | Perfluorohexane | 7.4 |

| | | |
|---|---|---|
| * - average from two measurements of 2.4 and 3.4 minutes respectively. | | |

### Examples 10-27

### Imaging of a dog heart using negatively charged perfluorobutane gas microbubbles and positively charged emulsions from Preparations 19-36

A 24 kg mongrel dog was anaesthetised, a mid-line sternotomy was performed, and the anterior pericardium was removed. Mid-line short-axis B-mode imaging of the heart was performed through a low-attenuating 30 mm silicone rubber spacer, using an ATL HDI-3000 scanner equipped with a P3-2 transducer. The framerate was 40 Hz and the mechanical index was 1.1. An amount of the perfluorobutane gas dispersion from Preparation 1 corresponding to 0.2 µl gas/kg body weight and an amount of perfluorodimethylcyclobutane emulsion corresponding to 0.02 µl perfluorodimethylcyclobutane/kg body weight were simultaneously injected intravenously into the dog when examining contrast agents comprising emulsions according to Preparations 19-28. For contrast agents comprising emulsions from Preparations 29-36 the corresponding doses were 0.35 and 0.04 µl/ml of gas and perfluorodimethylcyclobutane respectively. A substantial rise in echo intensity from the myocardium was seen, starting 20 seconds after the injection and lasting for 10 minutes; in each case the peak intensity was above that observed in Example 1(a).

Ultrasound opacification in the myocardium approximately 2 minutes after injection was corrected for baseline and the resulting myocardial contrast enhancements (MCEs) are given in the following Table 4. A substantial increase in myocardial opacification was seen at a time when the ventricles were almost emptied of contrast, indicating that the observed contrast enhancement is due to microbubbles retarded in the myocardium.

**Table 4**

| **Ex. No.** | **Cationic additive** | **Baseline-corrected MCE** |
|---|---|---|
| | | **(dB)** |
| 10 | DC-Cholesterol | 11.78 |
| 11 | 1,2-Distearoyl ethylphosphocholine | 17.03 |
| 12 | Benzylcetyldimethylammonium chloride | 10.43 |
| 13 | Cetyltrimethylammonium bromide | 11.46 |
| 14 | Cetylpyridinium chloride | 11.16 |
| 15 | Palmitoyl-Dpr(palmitoyl)-Arg-Arg-Lys-NH₂ | 10.64 |
| 16 | Myristoyl choline chloride | 10.29 |
| 17 | Hexadecanoic acid 2-aminoethyl ester | 14.41 |
| 18 | Hexadecanoic acid 4-aminobutyl amide | 12.74 |
| 19 | Aminoacetic acid hexadecyl ester | 16.14 |
| 20 | Cetyl carnitine ester | 12.11 |
| 21 | Psycosine | 13.56 |
| 22 | D-Sphingosine sulphate | 13.44 |
| 23 | Phytosphingosine | 13.56 |
| 24 | DL-Dihydrosphingosine | 17.05 |
| 25 | Didodecyldimethylammonium bromide | 9.54 |
| 26 | Methylaminoacetic acid hexadecyl ester | 15.50 |
| 27 | Dimethylaminoacetic acid hexadecyl ester | 15.33 |

### Example 28

### Imaging of a dog heart using negatively charged perfluorobutane gas dispersion and positively charged emulsion from Preparation 37

A 20 kg mongrel dog is anaesthetised, a mid-line sternotomy is performed, and the anterior pericardium is removed. Mid-line short-axis B-mode imaging of the heart is performed through a low-attenuating 30 mm silicone rubber spacer, using an ATL HDI-3000 scanner equipped with a P3-2 transducer. The framerate is 40 Hz and the mechanical index is 1.1. An amount of the perfluorobutane gas dispersion from Preparation 1 corresponding to 0.1 µl gas/kg body weight and an amount of the perfluorodimethylcyclobutane emulsion from Preparation 37 corresponding to 0.04 µl perfluorodimethylcyclobutane/kg body weight are simultaneously injected intravenously into the dog. The rise and duration of echo intensity from the myocardium are measured.

## Claims

1. A combined preparation for simultaneous, separate or sequential use as a contrast agent in ultrasound imaging, said preparation comprising:
i) a first composition which is an injectable aqueous medium comprising dispersed gas and material serving to stabilise said gas; and
ii) a second composition which is an injectable oil-in-water emulsion wherein the oil phase comprises a diffusible component capable of diffusion *in vivo* into said dispersed gas so as at least transiently to increase the size thereof, said composition further comprising material serving to stabilise said emulsion,
**characterised in that** material present at the surfaces of the dispersed gas phase and material present at the surfaces of the dispersed oil phase have affinity for each other,
with the proviso that when said first composition comprises biotinylated perfluorobutane microbubbles then said second composition does not comprise avidinylated perfluorodimethylcyclobutane emulsion droplets.

2. A combined preparation as claimed in claim 1 wherein the dispersed gas comprises air, nitrogen, oxygen, carbon dioxide, hydrogen, an inert gas, a sulphur fluoride, selenium hexafluoride, an optionally halogenated silane, an optionally halogenated low molecular weight hydrocarbon, a ketone, an ester or a mixture of any of the foregoing.

3. A combined preparation as claimed in claim 2 wherein the gas comprises sulphur hexafluoride or a perfluorocarbon.

4. A combined preparation as claimed in claim 3 wherein said perfluorocarbon is perfluoropropane, perfluorobutane or perfluoropentane.

5. A combined preparation as claimed in any of the preceding claims wherein the dispersed gas is stabilised by a coalescence-resistant surface membrane, a filmogenic protein, a polymer material, a non-polymeric and non-polymerisable wall-forming material or a surfactant.

6. A combined preparation as claimed in claim 5 wherein said surfactant comprises at least one phospholipid.

7. A combined preparation as claimed in claim 6 wherein at least 75% of said surfactant comprises phospholipid molecules individually bearing net overall charge.

8. A combined preparation as claimed in claim 7 wherein at least 75% of the surfactant comprises one or more phospholipids selected from phosphatidylserines, phosphatidylglycerols, phosphatidylinositols, phosphatidic acids and cardiolipins.

9. A combined preparation as claimed in claim 8 wherein at least 80% of said phospholipids comprise phosphatidylserines.

10. A combined preparation as claimed in any of the preceding claims wherein the diffusible component comprises an aliphatic ether, polycyclic oil, polycyclic alcohol, heterocyclic compound, aliphatic hydrocarbon, cycloaliphatic hydrocarbon or halogenated low molecular weight hydrocarbon, or a mixture of any of the foregoing.

11. A combined preparation as claimed in claim 10 wherein the diffusible component comprises one or more perfluorocarbons.

12. A combined preparation as claimed in claim 11 wherein said perfluorocarbon(s) comprise one or more perfluoroalkanes, perfluoroalkenes, perfluorocycloalkanes, perfluorocycloalkenes and/or perfluorinated alcohols.

13. A combined preparation as claimed in claim 12 wherein the diffusible component comprises one or more perfluoropentanes, perfluorohexanes, perfluorodimethylcyclobutanes and/or perfluoromethylcyclopentanes.

14. A combined preparation as claimed in any of the preceding claims wherein the diffusible component emulsion is stabilised by a phospholipid or lipopeptide surfactant.

15. A combined preparation as claimed in any of the preceding claims wherein the first and second compositions respectively contain surface materials with opposite charges.

16. A combined preparation as claimed in claim 15 wherein the first composition contains anionic surface material and the second composition contains cationic surface material.

17. A combined preparation as claimed in claim 16 wherein said anionic material is a negatively charged phospholipid and said cationic material is a lipophilic quaternary ammonium salt, a lipophilic pyridinium salt, a lipophilic primary, secondary or tertiary amine, a fatty acid amide of an optionally substituted di- or tri-amine, a fatty alcohol ester of an amino acid or a positively charged phospholipid or lipopeptide.

18. A combined preparation as claimed in claim 17 wherein said cationic material is present as an additive to the stabilising material of the second composition.

19. A combined preparation as claimed in any of the preceding claims which further includes a vasodilator and/or vasoconstrictor drug.

20. A combined preparation as claimed in claim 19 wherein said vasodilator drug is adenosine.

21. A combined preparation as claimed in any of claims 1 to 18 which further includes a therapeutic agent.

22. A combined preparation as claimed in any of claims 1 to 18 which further includes contrast-enhancing moieties for an imaging modality other than ultrasound.

23. A combined preparation as claimed in any of the preceding claims for the use as contrast agent in ultrasound imaging.

24. A combined preparation as claimed in claim 23 wherein microbubble growth from the contrast agent is activated within the imaged subject by application of external activation.

25. A combined preparation as claimed in claim 24 wherein said external activation comprises ultrasound irradiation.

## Patentansprüche

1. Kombinierte Zubereitung für simultane, getrennte oder sequentielle Verwendung als ein Kontrastmittel in der Ultraschallbildgebung, wobei die Zubereitung umfasst:
i) eine erste Zusammensetzung, die ein injizierbares wässriges Medium ist, das dispergiertes Gas und Material umfasst, das zum Stabilisieren des Gases dient; und
ii) eine zweite Zusammensetzung, die eine injizierbare Öl-in-Wasser-Emulsion ist, wobei die Ölphase eine diffundierbare Komponente umfasst, die zur Diffusion *in vivo* in das dispergierte Gas fähig ist, um zumindest transient die Größe davon zu vergrößern, wobei die Zusammensetzung weiter umfasst ein Material, das zum Stabilisieren der Emulsion dient,
**dadurch gekennzeichnet, dass** Material, das an den Oberflächen der dispergierten Gasphase vorhanden ist, und Material, das an den Oberflächen der dispergierten Ölphase vorhanden ist, eine Affinität füreinander besitzen, mit der Maßgabe, dass, wenn die erste Zusammensetzung biotinylierte Perfluorbutan-Mikroblässchen umfasst, die zweite Zusammenfassung nicht avidinylierte Perfluordimethylcyclobutan-Emulsionströpfchen umfasst.

2. Kombinierte Zubereitung nach Anspruch 1, wobei das dispergierte Gas umfasst Luft, Stickstoff, Sauerstoff, Kohlendioxid, Wasserstoff, ein inertes Gas, ein Schwefelfluorid, Selenhexafluorid, ein gegebenenfalls halogeniertes Silan, ein gegebenenfalls halogenierter Kohlenwasserstoff niedrigen Molekulargewichts, ein Keton, ein Ester oder eine Mischung von beliebigen der vorstehenden.

3. Kombinierte Zubereitung nach Anspruch 2, wobei das Gas umfasst Schwefelhexafluorid oder einen Perfluorkohlenwasserstoff.

4. Kombiniete Zubereitung nach Anspruch 3, wobei der Perfluorkohlenwasserstoff ist Perfluorpropan, Perfluorbutan oder Perfluorpentan.

5. Kombinierte Zubereitung nach einem der vorstehenden Ansprüche, wobei das dispergierte Gas stabilisiert ist durch eine koaleszenz-resistente Oberflächenmembran, ein filmogenes Protein, ein Polymermaterial, ein nichtpolymeres und nicht-polymerisierbares wand-bildendes Material oder ein oberflächenaktives Mittel.

6. Kombinierte Zubereitung nach Anspruch 5, wobei das oberflächenaktive Mittel mindestens ein Phospholipid umfasst.

7. Kombinierte Zubereitung nach Anspruch 6, wobei mindestens 75% des oberflächenaktiven Mittels Phospholipidmoleküle umfasst, die individuell eine Netto-Gesamtladung tragen.

8. Kombinierte Zubereitung nach Anspruch 7, wobei mindestens 75% des oberflächenaktiven Mittels ein oder mehrere Phospholipide umfasst, ausgewählt aus Phosphatidylserinen, Phosphatidylglycerolen, Phosphatidylinositolen, Phosphatidsäuren und Cardiolipinen.

9. Kombinierte Zubereitung nach Anspruch 8, wobei mindestens 80% des Phospholipids Phosphatidylserine umfassen.

10. Kombinierte Zubereitung nach einem der vorstehenden Ansprüche, wobei die diffundierbare Komponente umfasst ein aliphatisches Ester, polycyclisches Öl, polycyclischen Alkohol, heterocyclische Verbindung, aliphatischen Kohlenwasserstoff, cycloaliphatischen Kohlenwasserstoff oder halogenierten Kohlenwasserstoff niedrigen Molekulargewichts, oder eine Mischung von beliebigen der Vorstehenden.

11. Kombinierte Zubereitung nach Anspruch 10, wobei die diffundierbare Komponente einen oder mehrere Perfluorkohlenwasserstoffe umfasst.

12. Kombinierte Zubereitung nach Anspruch 11, wobei der/die Perfluorkohlenwasserstoff(e) einen oder mehrere Perfluoralkane, Perfluoralkene, Perfluorcycloalkane, Perfluorcycloalkene und/oder perfluorierte Alkohole umfasst/umfassen.

13. Kombinierte Zubereitung nach Anspruch 12, wobei die diffundierbare Komponente umfasst einen oder mehrere Perfluorpentane, Perfluorhexan, Perfluordimethylcyclobutane und/oder Perfluormethylcyclopentane.

14. Kombinierte Zubereitung nach einem der vorstehenden Ansprüche, wobei die diffundierbare Komponentenemulsion stabilisiert ist durch ein oberflächenaktives Phospholipid oder Lipopeptid.

15. Kombinierte Zubereitung nach einem der vorstehenden Ansprüche, wobei die erste und zweite Zusammensetzung entsprechend Oberflächenmaterialien mit entgegengesetzen Ladungen enthalten.

16. Kombinierte Zubereitung nach Anspruch 15, wobei die erste Zusammensetzung ein anionisches Oberflächenmaterial enthält und die zweite Zusammensetzung ein kationisches Oberflächenmaterial enthält.

17. Kombinierte Zubereitung nach Anspruch 16, wobei das anionische Material ein negativ geladenes Phospholipid ist und das kationische Material ist ein lipophiles quaternäres Ammoniumsalz, ein lipophiles Pyridiniumsalz, ein lipophiles primäres, sekundäres oder tertiäres Amin, ein Fettsäureamid eines gegebenenfalls substituierten Di- oder Triamins, einen Fettalkoholester einer Aminosäure oder ein positiv geladenes Phospholipid oder Lipopeptid.

18. Kombinierte Zubereitung nach Anspruch 17, wobei das kationische Material vorhanden ist als ein Additiv zum stabilisierenden Material der zweiten Zusammensetzung.

19. Kombinierte Zubereitung nach einem der vorstehenden Ansprüche, weiter umfassend ein Vasodilatator- und/oder Vasokonstriktor-Arzneimittel.

20. Kombinierte Zubereitung nach Anspruch 19, wobei das Vasodilatator-Arzneimittel Adenosin ist.

21. Kombinierte Zubereitung nach einem der Ansprüche 1 bis 18, weiter umfassend ein therapeutisches Mittel.

22. Kombinierte Zubereitung nach einem der Ansprüche 1 bis 18, weiter umfassend kontrast-verstärkende Einheiten für eine andere Bildgebungsmodalität als Ultraschall.

23. Kombinierte Zubereitung nach einem der vorstehenden Ansprüche zur Verwendung als Kontrastmittel in der Ultraschallbildgebung.

24. Kombinierte Zubereitung nach Anspruch 23, wobei das Mikrobläschen-Wachstum vom Kontrastmittel aktiviert ist innerhalb des abgebildeten Subjektes durch Anwendung einer externen Aktivierung.

25. Kombinierte Zubereitung nach Anspruch 24, wobei die externe Aktivierung Ultraschallbestrahlung umfasst.

## Revendications

1. Préparation combinée à utiliser de manière simultanée, distincte ou séquentielle en tant qu'agent de contraste en imagerie par ultrasons, ladite préparation comprenant :
(i) une première composition sous forme de milieu aqueux pouvant être injecté comprenant du gaz dispersé et un matériau servant à stabiliser ledit gaz ; et
(ii) une deuxième composition sous forme d'émulsion huile dans l'eau pouvant être injectée, dans laquelle la phase huileuse comprend un composant diffusible pouvant se diffuser in vivo dans ledit gaz dispersé de manière à augmenter la taille de celui-ci de façon au moins transitoire, ladite composition comprenant en outre un matériau servant à stabiliser ladite émulsion,
**caractérisée en ce que** le matériau présent aux surfaces de la phase gazeuse dispersée et le matériau présent aux surfaces de la phase huileuse dispersée ont des affinités,
à la condition que, lorsque ladite première composition comprend des microbulles de perfluorobutane biotinylées, ladite deuxième composition ne comprend pas de gouttelettes d'émulsion de perfluorodiméthylcyclobutane avidinylées.

2. Préparation combinée selon la revendication 1, dans laquelle le gaz dispersé comprend de l'air, de l'azote, de l'oxygène, du dioxyde de carbone, de l'hydrogène, un gaz inerte, un fluorure de soufre, un hexafluorure de sélénium, un silane éventuellement halogéné, un hydrocarbure de faible poids moléculaire éventuellement halogéné, une cétone, un ester ou un mélange des composés quelconques qui précèdent.

3. Préparation combinée selon la revendication 2, dans laquelle le gaz comprend un hexafluorure de soufre ou un perfluorocarbone.

4. Préparation combinée selon la revendication 3, dans laquelle ledit perfluorocarbone est le perfluoropropane, le perfluorobutane ou le perfluoropentane.

5. Préparation combinée selon l'une quelconque des revendications précédentes, dans laquelle le gaz dispersé est stabilisé par une membrane de surface résistante à la coalescence, une protéine filmogène, un matériau polymère, un matériau formant paroi non polymère et non polymérisable ou un agent de surface.

6. Préparation combinée selon la revendication 5, dans laquelle ledit agent de surface comprend au moins un phospholipide.

7. Préparation combinée selon la revendication 6, dans laquelle au moins 75 % dudit agent de surface comprend des molécules phospholipides portant chacune une charge globale nette.

8. Préparation combinée selon la revendication 7, dans laquelle au moins 75 % de l'agent de surface comprend un ou plusieurs phospholipides choisis parmi des phosphatidylsérines, phosphatidylglycérols, phosphatidylinositols, acides phosphatidiques et cardiolipines.

9. Préparation combinée selon la revendication 8, dans laquelle au moins 80 % desdits phospholipides comprennent des phosphatidylsérines.

10. Préparation combinée selon l'une quelconque des revendications précédentes, dans laquelle le composant diffusible comprend un éther aliphatique, une huile polycyclique, un alcool polycyclique, un composé hétérocyclique, un hydrocarbure aliphatique, un hydrocarbure cycloaliphatique ou un hydrocarbure de faible poids moléculaire halogéné, ou un mélange des composés quelconques qui précèdent.

11. Préparation combinée selon la revendication 10, dans laquelle le composant diffusible comprend un ou plusieurs perfluorocarbones.

12. Préparation combinée selon la revendication 11, dans laquelle le ou lesdits perfluorocarbones comprennent un ou plusieurs perfluoroalcanes, perfluoroalcènes, perfluorocycloalcanes, perfluorocycloalcènes et/ou alcools perfluorés.

13. Préparation combinée selon la revendication 12, dans laquelle le composant diffusible comprend un ou plusieurs perfluoropentanes, perfluorohexanes, perfluorodiméthylcyclobutanes et/ou perfluorométhylcyclopentanes.

14. Préparation combinée selon l'une quelconque des revendications précédentes, dans laquelle l'émulsion de composant diffusible est stabilisée par un agent de surface phospholipide ou lipopeptide.

15. Préparation combinée selon l'une quelconque des revendications précédentes, dans laquelle les première et deuxième compositions contiennent respectivement des matériaux de surface présentant des charges opposées.

16. Préparation combinée selon la revendication 15, dans laquelle la première composition contient un matériau de surface anionique et la deuxième composition contient un matériau de surface cationique.

17. Préparation combinée selon la revendication 16, dans laquelle ledit matériau anionique est un phospholipide chargé négativement et ledit matériau cationique est un sel d'ammonium quaternaire lipophile, un sel de pyridinium lipophile, une amine lipophile primaire, secondaire ou tertiaire, une amine d'acide gras ou une di- ou triamine éventuellement substituée, un ester d'alcool gras d'un acide aminé ou un phospolipide ou lipopeptide chargé positivement.

18. Préparation combinée selon la revendication 17, dans laquelle ledit matériau cationique est présent sous la forme d'un adjuvant au matériau stabilisateur de la deuxième composition.

19. Préparation combinée selon l'une quelconque des revendications précédentes, qui comprend en outre un médicament vasodilatateur et/ou vasoconstricteur.

20. Préparation combinée selon la revendication 19, dans laquelle ledit médicament vasodilatateur est de l'adénosine.

21. Préparation combinée selon l'une quelconque des revendications 1 à 18, qui comprend en outre un agent thérapeutique.

22. Préparation combinée selon l'une quelconque des revendications 1 à 18, qui comprend en outre des résidus d'amélioration de contraste pour une modalité d'imagerie autre que les ultrasons.

23. Préparation combinée selon l'une quelconque des revendications précédentes, à utiliser en tant qu'agent de contraste en imagerie par ultrasons.

24. Préparation combinée selon la revendication 23, dans laquelle la croissance des microbulles de l'agent de contraste est activée dans le sujet observé par l'application d'une activation externe.

25. Préparation combinée selon la revendication 24, dans laquelle ladite activation externe comprend une irradiation par ultrasons.
